# EUROPÄISCHE PATENTANMELDUNG

(11) **EP 1 736 148 A1**
(43) Veröffentlichungstag der Anmeldung: **27.12.2006**
(21) Anmeldenummer: 05726945.8
(22) Anmeldetag: 07.02.2005
(51) Int. Cl.: A61K 31/02, A61K 9/107, A61P 7/00, A61P 7/08

(54) **MEDIZINISCHE EMULSION VON PERFLUORORGANISCHEN VERBINDUNGEN UND HERSTELLUNGSVERFAHREN DAFÜR**

(30) Priorität: 01.03.2004 RU 2004106722
(71) Anmelder: Kuznetsova, Irina Nikolaievna, St. Petersburg 191036 (RU); Maievsky, Evgeny Ilich, Moskovskaya obl. 142290 (RU); Germanov, Evgeny Pavlovich, Moscow, 121085 (RU)
(72) Erfinder: KUZNETSOVA, Irina Nikolaievna, St.Petersburg, 191036 (RU); MAIEVSKY, Evgeny Ilich, Moskovskaya obl., 142290 (RU)
(74) Vertreter: Jeck, Anton
(86) Internationale Anmeldenummer: PCT/RU2005/000058
(87) Internationale Veröffentlichungsnummer: WO 2005/089739

(57) **Zusammenfassung**

Die Erfindung betrifft die Medizin, insbesondere Arzneimittel zur Behandlung von Blutverlusten, Hypoxie- und Ischämiezuständen sowie zur Verbesserung des Blutsauerstofftransports und der Erhaltung isolierter, perfusierender Organe und Gewebe. Die medizinische Emulsion gemäß der Erfindung aus perfluororganischen Verbindungen weist schnellausscheidbare, perfluororganischen Verbindungen, wie Perfluordekalin und Perfluoroctylbromid, einen Fluorkohlenstoffzusatz in Form einer Mischung aus perfluorierten, tertiären Aminen und Phospholipide in Form einer Wasser-Salz-Dispersion auf. Das Perfluordekalin und das Perfluoroctylbromid sind in der Zusammensetzung der schnellausscheidbaren, perfluororganischen Verbindungen in einem Verhältnis im Bereich von 10:1 bis 1:10 enthalten. Die Mischung der perfluorierten, tertiären Aminen hat die Form einer Mischung aus Perfluorotripropylamin und dessen Koprodukten: die cis- und trans-Isomere Perfluor-1-propyl-3,4-dimethylpirrolidon und Perfluor-1-propyl-4-methylpiperidin. Das Verfahren gemäß der Erfindung zur Herstellung der Emulsion umfasst die Herstellung der Wasser-Salz-Dispersion der Phopholipide, die Homogenisierung der perfluororganischen Verbindungen in der Dispersion bei hohem Druck und die gehörige Sterilisierung der Endemulsion. Die Haltbarkeitsdauer der Emulsion gemäß der Erfindung im nicht gefrorenen Zustand der Emulsion bei einer Temperatur von +4° C beträgt mindestens sechs Monate; in dieser Zeit wird die Biokompabilität dieser Emulsion in einem biologischen Medium (Blut, Plasma oder Serum) aufrecht erhalten.

## Beschreibung

Die Erfindung gehört zum Bereich der Biophysik und Medizin, insbesondere zu Arzneimitteln zur Behandlung von Blutverlusten, Hypoxie- und Ischämiezuständen sowie zur Verbesserung des Blutsauerstofftransports und der Erhaltung isolierter, perfusierender Organe und Gewebe.

### Liste der Abkürzungen, Bezeichnungen, Einheiten und Begriffe

- Tenside: grenzflächenaktive Substanzen
- P-268, F-268: Proxanol 268, Pluronic 268
- PFD: Perfluordekalin
- PFMHP: Perfluormethylzyklohexylpiperidin
- PFOB: Perfluoroktylbromid
- Fl: organische Flüssigkeit, die ein Gemisch aus Perfluortripropylamin und seinen Koprodukten, cis- und trans-Isomeren: Perfluor-1-propyl-3,4-dimethylpyrrolidon und Perfluor-1-propyl-4-methylpiperidin, darstellt.
- PFCs: Fluorkohlenstoffe, Fluorkohlenstoffverbindungen
- PFTBA: Perfluortributylamin
- PFTPA (PAF-3): Perfluortripropylamin
- Soja-P: Sojaphospholipide
- Ei-P: Eiphospholipide
- n: Wellenlängenexponent
- Cv: volumetrischer Gehalt an Fluorkohlenstoffen in Emulsion (ml/dl)
- a: mittlere Teilchengröße
- λ: Wellenlänge
- Ip: Reaktogenitätsindex

Der Erfolg in der Entwicklung von Infusionsmilieus, die Emulsionen aus Fluorkohlenstoffverbindungen enthalten, hängt größtenteils von den physikalisch-chemischen Eigenschaften ausgewählter PFCs und Emulsionen auf der Basis dieser PFCs sowie vom Herstellungsverfahren ab.

PFCs für medizinische Zwecke stellen Fluorkohlenstoffverbindungen unterschiedlicher Klassen dar. Äußerlich sind das klare, farb- und geruchlose Flüssigkeiten mit sehr hoher Dichte, etwa zweimal schwerer als Wasser. Eine ungewöhnlich starke C-F-Bindung (485,6 KJ/mol) führt dazu, dass die intermolekularen Kräfte dieser Verbindungen sehr schwach sind. Schwache intermolekulare Kräfte äußern sich in ihrer anormal starken Fähigkeit, Gase, darunter auch Blutgase, zu lösen.

Die PFCs sind aufgrund der starken C-F-Bindung durch eine chemische Inaktivität gekennzeichnet. Sie lösen sich schwer in Wasser und unterliegen dem Metabolismus im Organismus nicht. Die chemische Inaktivität der PFCs ist einer biologischen Inaktivität nicht gleich zu setzen. Bei intravenöser Injektion der Emulsionen auf PFC-Basis halten sich dies Emulsionen in Organen und Geweben, wobei die Verweilzeit von der PFC-Natur und der Emulsionsdosis abhängt.

Untersuchungen der biologischen Eigenschaften perfluorierter Verbindungen unterschiedlicher Klassen zeigten, dass die Ausscheidungsgeschwindigkeit von einer Reihe zusammenhängender, physikalisch-chemischer Parameter abhängt, nämlich von der Struktur und dem Molekulargewicht, der Siedetemperatur, dem Dampfdruck und der kritischen Lösungstemperatur in Hexan (*T_{kritisch}*)*. T_{kritisch}* ist diejenige Temperatur, bei der sich gleiche Volumen der zu untersuchenden Verbindung und von Hexan mischen. T_{kritisch} wird als Maß der relativen PFC-Lösungsfähigkeit in Lipiden betrachtet, das die Durchlaufgeschwindigkeit in Membranen kennzeichnet. Je niedriger T_{kritisch} ist, desto besser löst sich die Verbindung in Lipiden und desto schneller wird sie aus dem Organismus ausgeschieden. In der Tabelle 1 sind physikalisch-chemische Parameter angegeben, die als Auswahlkriterien von PFCs für die medizinische Anwendung dienen.

**Tabelle 1**

| **Werte für die kritische Lösungstemperatur in Hexan (T_{kritisch}), den Dampfdruck (P) und die Halbzerfallzeit (t_{1/2}) unterschiedlicher Verbindungen [1].** | | | | |
|---|---|---|---|---|
| Perfluorierte Verbindungen | Molargewicht | T_{kritisch} | P, mm QS (37°) | Halbzerfallzeit t_{1/2} 24 Stunden |
| Bizyklo[4.3.0]nonan | 412 | 13 | 33 | 4 |
| Dekalin | 462 | 22 | 12 | 7 |
| Dekahydroazenafphthen | 524 | 24 | 2 | 7 |
| N-(4-Methylzyklohexylpiperidin) | 595 | 38 | 1 | 60 (90) |
| 1-Propyl-2-methylpiperidin | 483 | 35 | 19 | 24 |
| Tripropylamin | 521 | 43 | 17 | 65 |
| Tributylamin | 671 | 61 | 1 | 900 |
| Dihexylether | 652 | 59 | 2 | 500 |

Aus den obigen Daten wird eine starke Korrelation zwischen T_{kritisch} und t_{1/2} sichtbar. Für den Dampfdruck wird diese Korrelation nicht beobachtet. Im hohen Maße hängen T_{kritisch} und das Molekulargewicht miteinander zusammen. Ein optimales Molekulargewicht für PFCs ist der Bereich zwischen 460 und 520. Insgesamt widersprechen alle angebotenen Auswahlkriterien für medizinische PFCs einander nicht, haben aber einen qualitativen Charakter. Heutzutage richten Forscher, die sich mit der Entwicklung und Untersuchung von Perfluorkohlenstoffemulsionen beschäftigen, ihr Interesse auf eine relativ begrenzte Anzahl von Verbindungen. In der Tabelle 2 und 3 sind die Strukturformeln und die physikalisch-chemischen Haupteigenschaften der am meisten verbreiteten PFCs angegeben.

**Tabelle 2**

| **Strukturformeln der am meisten verbreiteten und viel versprechenden PFV** | | |
|---|---|---|
| Perfluordekalin (PFD) Mol.Gew. 462 | Perfluortripropylamin (PFTPA) MoLGew. 521 | Perfluortributylamin (PFTBA) Mol.Gew. 671 |
| 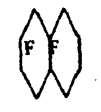 | 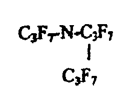 | 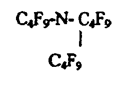 |
| Perfluortrimethylbizyklononan MoLGew. 562 | Perfluormethylisohinolin MöLGew. 495 | Perfluormethylzyklohexylpiperidin Mol.Gew. 595 |
| 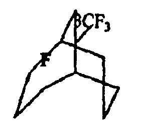 | 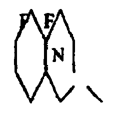 | 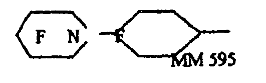 |
| Perfiluoroktylbromid (PFOB) CF₃-(CF₂)₆-CF₂Br Mol.Gew. 499 | Bis-Perfluorbutylethen (F-44E) C₄F₉-CH=CH-C₄F₉ Mol.Gew. 464 | Bis-Fluorhexylethen (F-66E) C₆F₁₃-CH=CH-C₆F₁₃ Mol.Gew. 664 |

Bei der Untersuchung primärer biologischer Eigenschaften unterschiedlicher PFCs wurde eine wichtige Anforderung formuliert: das Fehlen nicht identifizierbarer Beimischungen. Beimischungen mit unbekannten Eigenschaften können ein wahres Verhaltensbild (Verbleiben in Organen, Toxizität, Einfluss auf unterschiedliche Systeme des Organismus) der Grundsubstanz bei intravenöser Injektion verzerren.

**Tabelle 3**

| **Physikalisch-chemische Eigenschaften von PFCs, die als Basis für medizinische Präparate dienen.** | | | | | |
|---|---|---|---|---|---|
| Eigenschaften | PFD | PFTPA | PFMHP | PFOB | Perfluordezylbromid (PFDB) |
| Summenformel | C₁₀F₁₈ | C₉F₂₁N | C₁₂F₂₃N | C₈F₁₇Br | C₁₀F₂₁Br |
| Mol. Gewicht, g/mol | 462 | 521 | 595 | 499 | 599 |
| Siedetemperatur, °C | 142 | 131 | 168 | 143 | 180 |
| Dampfdruck, mm QS (37°C) | 12,7 | 18,0 | 2,0 | 10,5 | 1,5 |
| Kritische Lösungstemperatur, (T_{kritisch}) °C | 22 | 44 | 40 | -20 | 7 |
| Sauerstofflösbarkeit ml/100ml (Vol.-%), (37°C) | 40 | 45 | 40 | 53 | - |
| Halbzerfallzeit t_{1/2} | 7 | 65 | 90(60) | 4 | 40 |

Anmerkung: PFD/PFTPA sind die Basis für das Präparat Fluosol-DA; PFD/PFMHP für das Präparat Perftoran; PFOB/PFDB für das Präparat Oxygent.

Flüssige PFCs sind schlechte Lösungsmittel für unterschiedliche wasserlösbare, biologisch aktive Substanzen. Deswegen werden die PFCs zur Applikation als Sauerstofftransportmilieus in einer wässrigen Emulgatorlösung bis zur Gewinnung einer fein verteilten Emulsion dispergiert.

Die PFC-Fähigkeit, Gase auszutauschen, wird nach dem gesamten Sauerstoffgehalt in der Emulsion bestimmt. Die Sauerstoffkonzentration unterliegt dem Henry-Verteilungsgesetz und ist dem Sauerstoffdruck direkt proportional. Der Grundsatz der physikalischen Lösbarkeit der Gase in den PFCs erstreckt sich auch auf die Perfluorkohlenstoffemulsionen. Die in der Emulsion gelöste Sauerstoffmenge hängt von der Fluorkohlenstoffphase und nicht von der Teilchengröße ab, d. h. die in der Fluorkohlenstoffemulsion gelöste Sauerstoffmenge nähert sich den berechneten Werten durch Summieren der Gasmengenwerte jeder Phase (Sauerstoffmenge in der wässrigen Phase plus Sauerstoffmenge in den PFCs). Der Gehalt inerter Gase im Gemisch aus PFC und Plasma unterliegt auch dem Summengesetz der Gasmenge jeder Phase. Somit kann der Gehalt jedes Gases in der Emulsion nach physikalischen Gesetzen ihrer Lösbarkeit aufgrund des partiellen Gasdrucks und Volumenverhältnisses der Fraktionen PFC/H₂O berechnet werden. Dies bedeutet, dass der Sauerstoffgehalt in Perfluorkohlenstoffemulsionen umso höher wird, je höher sein partieller Druck oder seine Spannung (pO₂) und der Anteil der Fluorkohlenstoffphase sind.

Die spezifische (funktionelle) Wirkung jedes Präparats bei der Injizierung in den Körper wird durch die Präparatverträglichkeit, die durch den LD₅₀-Wert bestimmt wird, sowie durch das Fehlen von Nebenwirkungen, die sich hauptsächlich als Reaktogenität zeigen, bestimmt. Die Größe des LD₅₀-Werts für PFC-Emulsionen hängt stark von der Teilchengröße ab. Die mittlere Teilchengröße darf 0,2 µm nicht überschreiten. Die Vergrößerung des Anteils großer Teilchen (mittlere Größe über 0,4 µm) von 3 % bis 10 % reduziert den LD₅₀-Wert für die genannten Emulsionen um das Doppelte. Das Erkennen einer eventuellen Reaktogenität der Perfluorkohlenstoffemulsionen ist eines der schwierigsten Probleme, das bei der Entwicklung einer Arzneimittelform auf der Basis der Perfluorkohlenstoffemulsionen für eine intravenöse Injektion zu lösen ist. Bei der Anwendung eines Reaktogenitätspräparats kann sich beim Menschen eine allergische Reaktion entwickeln, die sich in unterschiedlicher Weise zeigt, von leichter Hautrötung bis zu einer anaphylaktoiden Reaktion mit Atem- und Herzstillstand.

Die meisten Forscher meinen, dass die Reaktogenität meistenteils von der Natur des Emulgators abhängt, der zum Dispergieren der Fluorkohlenstoffbasis der Emulsion eingesetzt wird und der eine (oberflächliche) Absorptionsschicht um die Teilchen bildet. Feste Meinung ist, dass die Reaktogenität der Emulsionen erster Generation von dem nichtionigenen Blockkopolymer von Oxyethylen und Polyoxypropylen, Pluronic F 68 (F-68), verursacht wurde und dessen Austausch durch natürliche Phospholipide das Reaktogenitätsproblem vollständig löst. Diese Meinung ist nicht ganz richtig, da Fettemulsionen trotz Stabilisierung durch natürliche Phospholipide eine Reaktogenität besitzen. Die Reaktogenität der Perfluorkohlenstoffemulsionen kann einfach durch den Einsatz von Phospholipiden als Emulgator und Stabilisator nicht beseitigt werden. Tatsächlich stellte es sich heraus, dass die Reaktogenität der PFC-Emulsionen vor allem durch die Oberflächeneigenschaften der emulgierten Teilchen bewirkt wird, d. h. durch den Zustand der Emulgatorschicht, die die Teilchen stabilisiert. Neben der chemischen Struktur, der Natur der Tensidmoleküle und den Schlüsselparametern, die sowohl die Stabilität des dispersen Systems als auch eventuelle Nebenreaktionen bestimmen, spielen aber die Bindungsfestigkeit der Tenside mit dem Ölkern der Emulsionsteilchen, der Lage der Moleküle auf der Oberfläche, die Dichte ihrer Packung, die Ausprägung der Absorptionseigenschaften, bezogen auf Proteine und andere biologisch aktive Moleküle, die sich im Blutfluss befinden, und schließlich die Größe der Emulsionteilchen eine Rolle. Der letzte Parameter soll besonders erwähnt werden. Eine Abnahme der mittleren Teilchengröße der Emulsion im Präparat Perpftoran, das nur durch das Blockkopolymer Polyoxyethylen und Polyoxypropylen, Proxanol 268, das der am nächsten kommende Prototyp von F-68 ist, stabilisiert wurde, verhalf zu einer raschen Verminderung der Nebenreaktion. Daraus geht klar hervor, dass oberflächliche Erscheinungen (Zusammenwirkung zweier Heterogensysteme, Emulsion und Blut bzw. Plasma) eine entscheidende Rolle im Verhalten der intravenös injizierten Emulsion bei der Entwicklung, Rezeptur und dem Herstellungsverfahren der Emulsionen spielen. Hierbei sollte durch Versuch die Zusammensetzung des Ölkerns als auch das mit diesem zusammenwirkende Tenside ausgewählt sowie die Vertretbarkeit der eingesetzten Technologie geprüft werden.

Bei der Entwicklung der Perfluorkohlenstoffemulsion gemäß der Erfindung für medizinische Zwecke und des Herstellungsverfahrens wurde jede Rezeptur und jedes technologische Element nach der biologischen Wirkung mittels eines Animationsmodells untersucht. Es ist bekannt, dass sich die Reaktogenitätsreaktion der Kaninchen bei der Injektion von Perfluorkohlenstoffemulsionen durch eine rasche Abnahme der neutrophilen Leukozyten im Peripherieblut äußert. Bei der Bewertung einer möglichen Reaktogenität der Perfluorkohlenstoffemulsion wird in Versuchen ein Reaktogenitätsindex *lp* verwendet, der nach der Formel *Ip*=*Ck*/*Cv* berechnet wird, in der *Ck* und *Cv* Neutrophile in % zum Ausgangsniveau in der Kontroll- und Versuchsgruppe bezeichnen. Wenn *Ip* nach 5 und 20 Minuten geringer als 3 ist, ist die Reaktogenitätswahrscheinlichkeit minimal [3].

Es sind unterschiedliche Verfahren zur Herstellung von Perfluorkohlenstoffemulsionen bekannt. 01-in-Wasser-Emulsionen, zu denen die Perfluorkohlenstoff emulsionen gehören und bei denen die Fluorkohlenstoffbasis eine Ölphase ist, werden unter hohen Energiekosten hergestellt. Eine Verkleinerung der Ölphase wird mittels Ultraschall oder mechanisch durchgeführt.

Bei der Ultraschalleinwirkung wird ein Dispergieren durch Reißkräfte unter scharfer, lokaler Druckänderung durchgeführt, die durch zwei Ursachen bedingt ist. Erstens wechseln sich eine lokale Kompression und eine Ausdehnung in der Flüssigkeit beim Wellendurchgang ab. Zweitens wirkt eine Kavitation ein, d. h. eine Bildung und ein Zusammenfallen von Hohlräumen, die mit den in Wasser gelösten Gasen gefüllt sind. Die Energie und die Kraft der Ultraschalleinwirkung, die zur Herstellung einer Submikro-Emulsion benötigt werden, sind so groß, dass neben dem Dispergieren die C-F-Bindung gelöst wird. Dadurch erscheinen hochtoxische Konzentrationen der F⁻-Ionen, etwa 3-5 mmol, in der wässrigen Phase der Emulsion. Eine Emulsion mit einem solch hohen Gehalt von F⁻ ist für den Blutersatz sowie zur Erhaltung perfusierender Organe nicht brauchbar. Sie soll vom Überschuss der F⁻-Ionen mittels Hindurchleitung durch ein lonenaustauschharz befreit werden. Der zweite Nachteil einer mit Ultraschall dispergierten Emulsion besteht in einem außerordentlich hohen Dispersionsbereich, denn bei einer mittleren Teilchengröße von 0,1 µm kann ein großer Teilchenanteil in der Größe von über 0,4 µm und unter 0,01 µm vorgefunden werden.

Ein mechanisches Dispergieren durch Schütteln oder kräftiges Rühren lässt nur grob verteilte Emulsionen mit der zur biomedizinischen Anwendung nicht akzeptierbaren Teilchengröße über ein Millimeter entstehen. Zur Herstellung fein verteilter Emulsionen wird ein Durchdrücken der Substanz der dispersen Phase durch feine Löcher in das Dispersionsmittel unter hohem Druck (Extrusion) verwendet, wodurch der Flüssigkeitsstrahl in Tröpfchen gebrochen wird. Das Dispergieren wird durch den Druckgradienten und hydraulische Reibungskräfte bewirkt. Die Emulsionen werden gewöhnlich in Hochdruckhomogenisatoren hergestellt. Ein Stabilisieren der erhaltenen Emulsionen wird mit Hilfe grenzflächenaktiver Substanzen oder Emulgatoren erreicht. Die stabilisierende Wirkung dieser Substanzen wird durch zwei Ursachen erklärt: erstens durch den Abbau überflüssiger Oberflächenenergie zwischen den Phasen bzw. durch den Abbau der Oberflächenspannung und zweitens durch die Bildung einer strukturellen, mechanischen Barierre (Absorptionsschicht), die die Stabilität der Teilchen gewährleistet und den Kontakt oder Verklebung bzw. Verklumpung der Teilchen verhindert.

Unter vielen Tensiden erfüllen nur wenige die Anforderungen an die Anwendbarkeit zur Herstellung von Präparaten zu intravenöser Injektion (Tabelle 4).

**Tabelle 4**

| **Übliche grenzflächenaktive Substanzen zur Herstellung von Perfluorkohlenstoffemulsionen** | | |
|---|---|---|
| Bezeichnung | Strukturformel | Grundparameter |
| Proxanol 268 (Pluronic F-68) | 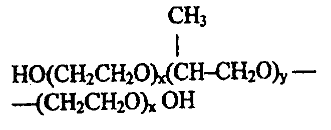 | Synthetisches Blockkopolymer, M.G. ~13000 (P-268) und ~9000 (F-68), x= Anzahl der Kettenglieder von Ethylenpolyoxid-Block, y= Anzahl der Kettenglieder von Propylenpolyoxid-Block. Gut wasserlöslich. |
| Phospholipide | 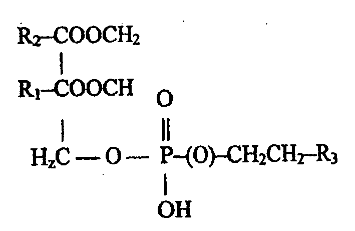 | Natürliche Verbindung. R₁ und R₂ sind unterschiedliche Ketten der Fettsäuren. |
| (Eigelb-)Lezithin | R₃=N(CH₃)₃ | M.G. 760-870. In Wasser praktisch unlöslich |

Zurzeit werden hauptsächlich zwei Emulgatoren zur Herstellung von Perfluorkohlenstoffemulsionen, nämlich Proxanol-268 (Pluronic F-68) und natürliche Phospholipide (Ei- und Sojaphospholipide usw.), eingesetzt.

Die Proxanol-Struktur stimmt mit den charakteristischen Moleküleigenschaften wasserlöslicher Tenside nicht überein, die einen polaren Kopf (hydrophilen Teil) und einen unpolaren Schwanz (hydrophoben Teil) haben. Bei Proxanol wird der hydrophile Molekülcharakter von zwei Polyoxidethylen-Ketten bestimmt, wobei Wasserstoffbindungen mit H₂O-Molekülen gebildet werden. Methylgruppen von Polypropylenpolyoxid setzen lipophile Eigenschaften seines Moleküls voraus. Das Verhältnis der Blöcke Polyoxidethylen/Polyoxidpolypropylen für F-68 und P-268 ist durchschnittlich gleich und beträgt 80:20. Die stabilisierende Wirkung dieser Emulgatoren wird hauptsächlich durch die sterische Wirkung des Schutzfilms bewirkt, der von den grenzflächenaktiven Molekülen um die Fluorkohlenstoffteilchen gebildet wird. Dabei bildet der größte Teil Tensidmoleküle neben den in der Absorptionsschicht gebundenen Tensiden verschiedene mizellare Strukturen in der wässrigen Phase, darunter auch die, die von Fluorkohlenstoffverbindungen frei sind. Zwischen den Tensidemolekülen in der Absorptionsschicht und in den Mizellen der wässrigen Phase tritt ein dynamisches Gleichgewicht ein, das einerseits zur Stabilisierung der Absorptionsschicht benötigt wird und andererseits die Dichte der Molekülpackung der Tenside in der Absorptionsschicht bei Dauerlagerung stört.

Die Phospholipide stellen ein Gemisch aus Verbindungen natürlicher Herkunft dar, deren allgemeine Struktur in der Tabelle 4 angegeben ist. Phospholipide sind wasserunlöslich und keine guten lipophilen Wirkstoffe in Bezug auf unterschiedliche Fluorkohlenstoff-Verbindungen gleichzeitig, obwohl sie durch PFD und PFTPA in der Doppelschicht der Phosphatidylcholinteilchen teilweise gelöst werden. Die Zusammenwirkung der Phospholipide und Fluorkohlenstoffverbindungen in der wässrigen Phase hat einen doppelten Charakter. Es ist möglich, Fluorkohlenstoffverbindungen in die Lamellenstrukturen der Phospholipide einzuschließen und (oder) Monoschichten der Phospholipide zu bilden, die mit der Teilchenoberfläche irreversibel verbunden sind. Möglich sind nicht homogene Teilchen in Emulsionen aus Fluorkohlenstoffverbindungen und Phospholipiden, d. h. Teilchen, die mit einer Schutzschicht aus Phospholipiden umhüllt und von Phospholipiden frei sind. Diese Nichthomogenität kann auf verfahrenstechnische Besonderheiten und/oder Phospholipidüberschuss in Bezug auf die Fluorkohlenstoffphase zurückgeführt werden.

Für fein verteilte Emulsionen ist der bestimmende Mechanismus der Feinheitssenkung (Teilchenvergröberung) eine isothermische oder molekulare Substanzdestillation der dispersen Phase von kleinen zu größeren Teilchen mittels einer Diffusion der Moleküle von Fluorkohlenstoffverbindungen durch ein Dispersionsmedium. Dieser Prozess heißt "Ostwald-Reifung" der Emulsion oder "Umkondensation". Die treibende Kraft dieses Prozesses ist ein größerer Druck von gesättigtem Dampf über kleinere Teilchen im Vergleich zu größeren. In diesem Fall ist ein wichtiger Parameter auch der Lösbarkeitsgrad von Fluorkohlenstoffverbindungen im wässrigen Milieu. Eine Verhinderung der Umkondensation kann eine entscheidende Bedeutung zur Erhaltung eines beständigen Aggregatzustands der Perfluorkohlenstoffemulsionen, d. h. die Erhaltung der Feinheit und Eigenartigkeit der Teilchen, haben. Die Hauptwege der Destabilisierung, nämlich eine molekulare Diffusion und eine weniger bedeutende Flockung und Koagulation, sind sowohl für relativ verdünnte Emulsionen, in denen die Fluorkohlenstoffphase unter 20 Vol.-% liegt, als auch für höher konzentrierte Emulsionen, in denen Fluorkohlenstoffphase 50 Vol.-% beträgt, charakteristisch.

Die Stabilisierungswege der Perfluorkohlenstoffemulsionen sind bekannt. Das Stabilisierungsgrundprinzip von Kolloidsystemen heißt Verhinderung ihrer Zerfallmechanismen. Eine Zugabe von Zucker und Koemulgatoren mit negativer Ladung (Minoritätskomponenten der Phospholipide) in Emulsionen auf PFC/Phospholipid-Basis verhindert eine Flockung der Teilchen durch Änderung der räumlichen Wechselwirkung der Tensidmoleküle in der Absorptionsschicht sowie durch eine Vergrößerung der elektrostatischen Abstoßungskraft zwischen den Teilchen.

Die Senkung des Hauptzerfallprozesses der Perfluorkohlenstoffemulsionen, die durch molekulare Diffusion verursacht wird, wird durch Zugabe einer zweiten weniger wasserlöslichen Komponenten (zusätzliche Fluorkohlenstoffverbindung) zur Fluorkohlenstoffbasis erzielt, die eine höhere Siedetemperatur besitzt und diesen Prozess verlangsamt.

Dieser Stabilisierungsgrundsatz wird in der Entwicklung der Präparate Fluosol-DA, Perpftoran und Oxygent eingesetzt. In der folgenden Tabelle 5 sind zusammengefasste Daten nach der Zusammensetzung und den physikalisch-chemischen Eigenschaften der genannten Präparate dargestellt.

**Tabelle 5**

| **Zusammengefasste Daten nach der Zusammensetzung der Präparate Fluosol-DA (Japan), Perftoran (Russland) und Oxygent (USA) /2/.** | | | | | |
|---|---|---|---|---|---|
| Inhaltsstoffe | Konzentration (Gew.-/Vol.-%) | | | | |
| | Fluosol-DA | Perftoran | Oxygent | | |
| | | | AF0104 | AF0143 | AF0144 |
| Perfluordekalin (PFD) | 14 | 13 | -- | -- | -- |
| Perfluortripropylamin (PFTPA) | 6 | -- | -- | -- | -- |
| Perfluormethylzyklohexylpiperidin (PFMHP) | -- | 6,5 | -- | -- | -- |
| Perfluoroktylbromid (PFOB) | -- | -- | 90 | 87 | 58 |
| Perfluordezylbromid (PFDB) | -- | -- | -- | 3 | 2 |
| Pluronic F-68 (Proxanol-268) | 2,72 | 4 | -- | -- | -- |
| Phospholipide | 0,4 | - | 4 | 5,4 | 3,6 |
| Kaliumoleat | 0,032 | -- | -- | -- | -- |
| Puffersubstanz | CO₃⁻² | CO₃⁻² | PO₄⁻³ | PO₄⁻³ | PO₄⁻³ |
| Bivalente Kationen | + | + | -- | -- | -- |

In den ersten zwei Präparaten werden als Zusätze die Fluorkohlenstoffverbindungen Perfluortripropylamin und Perfluormethylzyklohexanpiperidin, mit höherer Siedetemperatur und weniger wasserlöslich, zu Perfluordekalin, das den größten Anteil der Ölphase hat, gegeben. Als Emulgator wird wasserlösliches Pluronic F-68 mit Phospholipidzusatz (Fluosol-DA) oder sein Prototyp Proxanol-268 (Perftoran) eingesetzt. Nach ihren physikalisch-chemischen Eigenschaften unterscheiden sie wenig voneinander. Sie gehören zu Präparaten erster Generation, deren allgemeiner Nachteil darin besteht, dass sie wegen unzureichender Stabilität eingefroren zu lagern sind. Zu der Fluorkohlenstoffbasis von Oxygent (Perfluoroktylbromid) wird Perfluordezylbromid gegeben, das eine höhere Siedetemperatur hat und weniger wasserlöslich ist. Der Vorteil von Oxygent, das zur zweiten Generation gehört, wird durch Lagerung im nicht eingefrorenen Zustand bestimmt. Ferner wird Perfluoroktylbromid, das die Fluorkohlenstoffbasis des Präparats ist, schnell aus dem Organismus beinahe mit derselben Geschwindigkeit wie Perfluordekalin (*t*_{*1*/*2*} ~4 und 7 Tage entsprechend) ausgeschieden.

Oxygent ist eine kaufmännische Bezeichnung von Infusionsmedien, die sich hinsichtlich der Zusammensetzung etwas unterscheiden.

Der Emulgator trägt nicht nur zur Senkung der oberflächlichen Zwischenphasenspannung im System H₂O/PFC, die zur Feinheit benötigt wird, bei. Eine Änderung der Emulgatornatur kann die Geschwindigkeit der Molekulardiffusion beeinflussen. Für die Zukunft werden fluorierte Tenside für aussichtsreich gehalten, die in ihrem Molekül einen fluorierten, hydrophoben und einen nicht fluorierten, hydrophilen Teil enthalten. Große Erfolge in der Synthese fluorierter Tenside für Fluorkohlenstoffverbindungen erzielten in der letzten Zeit französische Chemiker [4]. Die allgemeine Struktur synthetischer, fluorierter Tenside stellt eine Kombination aus einer perfluorierten Kette und einem polaren Kopf dar. Als Bindungsglied dieser Elemente wird eine Kohlenwasserstoffkette verwendet. Der polare Kopf wird aus natürlichen Substanzen oder deren Derivaten ausgewählt. Fluorierte Tenside, die als polaren Kopf Alkohole oder Zuckerderivate enthalten, weisen eine Synergie mit Pluronic F-68 auf. Der Einsatz von Phospholipiden, Zuckerphosphaten oder Phosphatidylcholin in fluorierten Tensiden als polarer Kopf erhöhen die Stabilität der Fluorkohlenstoffemulsionen, die natürliche Phospholipide als Emulgatoren enthalten. Es wurde eine neue Klasse gemischter, fluorierter Tenside zur Stabilisierung vorgeschlagen [4]. Die Moleküle dieser Klasse fluorierter Tenside stellen einen Block aus zwei linearen Bestandteilen dar, nämlich einen Kohlenwasserstoffbestandteil und einen perfluorierten Bestandteil. Die allgemeine Formel dieser Verbindungen lautet folgendermaßen:

CₙF₂ₙ₊₁CₘH₂ₘ₊₁ oder CₙF₂ₙ₊₁CH=CHCₘH₂ₘ₊₁

Die Erfinder nennen diese Moleküle "dowel", was buchstäblich "Feder" oder "Verbindungselement" bedeutet.

Es herrscht die Meinung, dass Moleküle fluorierter Tenside mit einem allgemeinen, linearen RH-RF-Aufbau die Rolle eines Festigungselements spielen, dessen Kohlenwasserstoffende in den Lipidfilm, der Pefluorkohlenstoffteilchen umhüllt, und dessen anderes, fluoriertes Ende in die Ölphase hineingeht, d. h. dass die RH-RF-Moleküle die Adhäsionseigenschaften der Tensidoberflächenschicht verbessern.

Heutzutage sind Perfluordekalin und Perfluoroktylbromid die am meisten akzeptierten Verbindungen zur Herstellung biomedizinischer Emulsionen deswegen, weil sie schnell aus dem Organismus im Vergleich zu anderen Fluorkohlenstoffverbindungen ausgeschieden werden.

Bekannt sind Patente [5, 6], in denen Zusammensetzungen von Blutersatzmitteln beschrieben werden, deren Fluorkohlenstoffbasis Gemische aus zwei (Perfluordekalin/Perfluormethylzyklohexilpiperidin oder Perfluordekalin/Perfluortributylamin oder Perfluoroktylbromid/Perfluormethylzyklohexilpiperidin), aus drei (Perfluoroktylbromid/Perfluordekalin/Perfluormethylzyklohexilpiperidin oder Perfluoroktylbromid/Perfluordekalin/Perfluortributylamin) oder sogar aus vier Fluorkohlenstoffverbindungen (Perfluoroktylbromid/Perfluordekalin/Perfluormethylzyklohexilpiperidin/Perfluortributylamin) in verschiedenem Verhältnis darstellen. Diese Gemische dispergieren mittels des wasserlöslichen Emulgators Proxanol P-268. Der Einsatz dieses Emulgators ermöglicht nicht, die genannten Gemische bei positiven Temperaturen zu lagern. Darüber hinaus haben diese Emulsionen nach dem Auftauen eine begrenzte Lagerdauer bei +4° C (höchstens 1 Monat). Das ist ihr Hauptnachteil.

Patentgeschützt sind Emulsionen mit fluorierten Tensiden. Die fluorierte Tenside [7] enthaltenden Mikroemulsionen finden zurzeit keine praktische Anwendung als Infusionsmedium eher deswegen, weil sie in vivo nicht ausreichend stabil sind. Bekannt ist eine andere Zusammensetzung von Perfluorkohlenstoffemulsionen, die mittels gemischter, fluorierter Tenside hergestellt werden, die im Molekül einen fluorphilen Teil und einen lipophilen Teil enthalten [8]. Diese Emulsionen behalten zwar den mittleren Teilchendurchschnitt bei positiven Temperaturen bei, aber nur innerhalb von 3 Monaten.

Bekannt ist ein Patent [9], bei dem als Phospholipidquelle zur Herstellung von Emulsionen eine 10 %ige Fettemulsion aus Liposyn dient. Als Fluorkohlenstoffbasis sind drei Gruppen Fluorkohlenstoffverbindungen patentgeschützt. Zur ersten Gruppe gehören Perfluorzykloalkane oder Perfluoralkylzykloalkane (darunter Perfluordekalin, Perfluormethyldekalin, Perfluorperhydrophenanthren u. a.). Die zweite Gruppe umfasst perfluoralkylgesättigte, heterozyklische Verbindungen. Die dritte Gruppe besteht aus perfluorierten, tertiären Aminen, und zwar Perfluortributylamin, Perfluortripropylamin u. a. Zu den anwendbaren Fluorkohlenstoffverbindungen gehört auch Perfluoroktylbromid. Dennoch gelingt es nicht, mit Hilfe des 10 %igen Liposyn eine stabile Perfluordekalin-Emulsion herzustellen. Ihre maximale Lagerdauer ist 25 Tage.

Bei einem weiteren Patent [10] werden Eiphospholipide zur Emulsionherstellung eingesetzt. Der Anteil der Fluorkohlenstoffphase ändert sich in einem großen Bereich von 10 bis 50 Vol.-% und der der Phospholipide von 0,5 bis 7 Gew.-%. Als Ölphase wird im Patent nur eines der PFCs aus der breiten Klasse der Verbindungen ausgewählt und eingesetzt, nämlich die Perfluorhydrophenanthren-Gruppe mit Fluoratomen von 1 bis 24, Perfluordekalin, Perfluoroktylbromid, Perfluormethyladamantan und Perfluorperhydrophenanthren.

Der Schwerpunkt in beiden genannten Patenten sind Verfahren zur Erhaltung unterschiedlicher Organe und Systeme durch den Einsatz hergestellter Fluorkohlenstoffemulsionen. Vor Beginn von physiologischen Versuche werden Emulsionen mit Kristalloidlösungen und/oder onkotonischen Wirkstoffen (Albumin, Hydroxyethylstärke) gemischt. Die vorgeschlagenen Emulsionen gehören zwar zu Emulsionen der zweiten Generation, haben aber einen wesentlichen Nachteil. In beiden Patenten werden Untersuchungsergebnisse der Emulsionstabilität, d. h. das Beibehalten der Teilchengröße bei Dauerlagerung (über einen Monat), nicht angegeben. Die beiden letztgenannten Patente [9, 10] werden hier als Prototype betrachtet.

Der der Emulsion gemäß der Erfindung am nächsten kommende Prototyp ist die unter [11] genannte Emulsion. Diese Emulsion, als Prototyp genommen, gehört zur zweiten Generation und enthält eine schnell ausscheidende Fluorkohlenstoffverbindung in der Menge von 40 bis 50 Vol.-% und einen perfluorierten Zusatz einer höher siedenden Verbindung von 5 bis 10 Vol.-%. Als schnell ausscheidende Fluorkohlenstoffverbindung wird Perfluordekalin oder Perfluoroktylbromid (Hauptkomponente) und als Zusatz Perfluormethylzyklohexylpiperidin eingesetzt. Der Emulgator ist Ei- bzw. Sojaphospholipid.

Das Perfluorzyklohexylpiperidin stabilisiert die Emulsion, verringert die Geschwindigkeit der Molekulardiffusion (Umkondensation) der Hauptkomponenten (Perfluordekalin bzw. Perfluoroctylbromid) und wird zur Herstellung von Emulsionen anderer Zusammensetzung, nämlich Perftoran, eingesetzt. Der Hauptnachteil der aus dem Patent [11] bekannten Emulsion ist ein relativ großer Teilchendurchschnitt über 0,2 µm.

Die Aufgabe dieser Erfindung besteht in der Erhöhung der Stabilität der Emulsion und in der Verbesserung der Emulsionsqualität, d. h. in der Erhaltung der Biokompatibilität mit biologischem Milieu (Blut, Plasma bzw. Serum) bei einer Lagerung von mindestens 6-12 Monaten im nicht eingefrorenen Zustand.

Die Emulsion gemäß der Erfindung für medizinische Zwecke enthält schnellausscheidendes Perfluordekalin bzw. Perfluoroktylbromid sowie einen perfluorierten Zusatz und ein Phospholipid. Diese Emulsion ist dadurch gekennzeichnet, dass als schnellausscheidende Komponente eine Zusammensetzung aus gemischten Perfluordekalin und Perfluoroktylbromid eingesetzt wird, dass der perfluorierte Zusatz ein Gemisch aus perfluorierten, tertiären Aminen darstellt und dass die Phospholipide als Dispersion im Wasser-Salz-Medium eingesetzt werden.

Die Emulsion ist weiter dadurch gekennzeichnet, dass die Gesamtkonzentration an Fluorkohlenstoffverbindungen im Bereich von 2 bis 40 Vol.-% liegt.

Die Emulsion ist ferner dadurch gekennzeichnet, dass die Zusammensetzung das schnellausscheidende Perfluordekalin und Perfluoroktylbromid im Verhältnis von 10:1 bis 1:10 enthält, dass der perfluorierte Zusatz 1 % bis 50% des Gesamtgehalts der Fluorkohlenstoffverbindungen ist und cis- und trans-Isomere von Perfluor-1-propyl-3,4-dimethylpirrolidon und Perfluor-1-propyl-4-methylpiperidin sowie zusätzliches Perfluor-N-methylcyclohexylpiperidin und dessen Koprodukte enthält.

Die Emulsion ist ferner dadurch gekennzeichnet, dass sie eine Dispersion der Eiund Soja-Phospholipide oder ein Gemisch aus diesen Lipiden im Wasser-Salz-Medium in der Konzentration von 0,2 bis 5 Gew.-% enthält.

Die Emulsion ist ferner dadurch gekennzeichnet, dass die Phospholipiddispersion im Wasser-Salz-Medium ein Adjuvans von 1 bis 15 % vom Gesamtgehalt der Phospolipide enthält. Als Adjuvans wird pflanzliches Öl eingesetzt, und zwar Soja-, Sonnenblumenkern- oder Rizinusöl bzw. ein Gemisch dieser Öle im wirksamen Verhältnis als doppeltes oder dreifaches Gemisch.

Die Emulsion ist ferner dadurch gekennzeichnet, dass das Wasser-Salz-Medium Natrium- und Kaliumsalze von Chloriden und Phosphaten sowie das Monosaccharid Mannitol im Injektionswasser enthält und die Konzentration der Komponenten im Wasser-Salz-Medium einen osmotischen Druck im Bereich von 100 bis 350 mosmol/l aufweist.

Die Emulsion ist ferner dadurch gekennzeichnet, dass der mittlere Teilchendurchschnitt 0,2 µm nicht überschreitet und im Bereich von 0,06-0,2 µm liegt.

Das Herstellungsverfahren der Emulsion gemäß der Erfindung mittels Homogenisierung ist dadurch gekennzeichnet, dass das Verfahren mehrere Stufen enthält, die eine Phospholipiddispersion im Wasser-Salz-Medium, ein Homogenisieren in der Phospholipiddispersion, eine Hitzesterilisation der fertigen Emulsion und eine nachfolgende Lagerung von mindestens 6 Monaten im nicht eingefrorenen Zustand bei +4° C umfassen.

Das Herstellungsverfahren gemäß der Erfindung ist ferner dadurch gekennzeichnet, dass die Phospholipiddispersion im Wasser-Salz-Medium durch Homogenisieren unter einem Hochdruck von mindestens 100 atm und mit einer nachfolgenden Sterilisation hergestellt wird.

Das Herstellungsverfahren gemäß der Erfindung ist ferner dadurch gekennzeichnet, dass die Fluorkohlenstoffverbindungen in der Phospholipiddispersion unter einem Druck von 300 bis 650 atm homogenisiert werden.

Das Herstellungsverfahren gemäß der Erfindung ist ferner dadurch gekennzeichnet, dass die Phospholipiddispersion und Emulsion bei einer Temperatur von 100° C sterilisiert werden.

Wie oben angegeben ist, besteht die Aufgabe der Erfindung in einer Erhöhung der Emulsionstabilität und in einer Verbesserung der Emulsionsqualität, d. h. in der Erhaltung der Biokompatibilität im biologischen Milieu (Blut, Plasma oder Serum) bei einer Lagerung von mindestens 6-12 Monaten im nicht eingefrorenen Zustand. Der Begriff Biokompatibilität umfasst unterschiedliche Werte und soll auf die Emulsion bezogen präzisiert werden. In den o. g. Patenten [8-11] wird unter Biokompatibilität eine relativ hohe Ausscheidegeschwindigkeit der gewählten PFCs, die Fähigkeit zur Erhaltung der Gewebe und Organe, durch die die Emulsion perfundiert wird, und eine vergleichbar niedrige Toxizität für Tiere (wenigstens 2 Volumen durchströmenden Bluts) verstanden. Diese Vorstellungen schließen einander nicht aus, spiegeln aber den ersten Schritt nicht wider, nämlich die Zusammenwirkung der Teilchen mit Plasma und Blut im Gefäßstrom. Bei der vorliegenden Erfindung beginnt die Biokompatibilität mit dem Ausprägungsgrad der Zusammenwirkung (Reaktion) der Emulsion mit dem biologischen Milieu (Blut, Plasma oder Serum). Die Ergebnisse dieser Zusammenwirkung können nicht nur in vivo, sondern auch vor allem bei Versuchen in vitro nach dem Stabilisierungsgrad der Emulsion bei dem Einfluss einer Reihe von Faktoren bewertet werden, die die Verletzung der Absorptionsschicht bei der Lagerung und dem Eindringen der Emulsion in den Blutstrom simulieren.

Die Qualität und Stabilität der Emulsionen wird üblicherweise aufgrund der Teilchengröße charakterisiert, und zwar soll der mittlere Teilchendurchschnitt 0,2-0,3 µm nicht überschreiten. Ein solcher Ansatz ist für biomedizinische, disperse Präparate zur intravenösen Injektion nicht ausreichend. Dies ist dadurch begründet, dass das Fluorkohlenstoffteilchen als Fremdmaterial mit Proteinen und Molekülen anderer, sich im Plasma befindlicher Verbindungen sowie mit Blutzellen beim Eindringen in den Blutstrom zusammenwirkt. Der allgemeine Charakter der Zusammenwirkung hängt von den Eigenschaften der Teilchenoberfläche ab. Die funktionelle Aktivität (Gastransportfunktion) der Emulsionen hängt wesentlich von der Kompatibilität der Oberfläche der emulgierten Teilchen mit Blut und Plasma ab, da z. B. bei der Systemaktivierung des Komplements auf der Fremdoberfläche eine Reaktionskaskade ausgelöst wird, die einen Gefäßkrampf und eine Störung des Regionärblutflusses verursacht. Es soll auch bemerkt werden, dass die Emulsionstabilität in vitro durch die Eigenschaften der Absorptionsschicht von Tensiden um die Teilchen (Festigkeit, Topographie der Oberfläche usw.) stark bedingt wird. Im Sinne des Gesagten kann das Problem der Emulsionstabilität nur mit Hilfe üblicher chemischer Kolloidmethoden der Teilchenuntersuchung ohne Bewertung der Strukturbesonderheiten nicht gelöst werden. Äußerst aktuell ist eine diesbezügliche Entwicklung einfacher Methoden und Ansätze, die Informationen über die Teilchengröße und Ganzheit der Teilchenstruktur liefern können. Dabei soll der Begriff Struktur selbst in Bezug auf Emulsionen präzisiert werden.

Fortschritte in der Untersuchung der Emulsionsstabilität in vitro und in vivo hängen mit der Erweiterung und Vertiefung des Begriffs Struktur sowie mit der Entwicklung der Untersuchungsmethoden der Struktur zusammen. Der Begriff Stabilität eines Präparats oder einer Substanz wird durch die Stabilität der Eigenschaften diverses Präparats oder dieser Substanz bestimmt. Die die Eigenschaften der Emulsion bestimmenden Parameter charakterisieren die Emulsionsstabilität nicht ausreichend. Bei diesseitigen Versuchen wurden die Vorstellungen über die Stabilität der Emulsionen mit Rücksicht auf Besonderheiten der Emulsionsstruktur erweitert.

Die Stabilität der Kohlenstoffemulsionen wird üblicherweise nach Änderung der Teilchengröße der Emulsion beim Lagern bewertet. Dieser rein chemische Kolloidansatz ist nicht ausreichend. Für Emulsionen, die die Basis für Präparate darstellen und zu intravenösen Injektionen verwendet werden sollen, gewinnt eine Information über die Emulsionsstabilität nicht nur bei Versuchen in vitro, sondern auch die Möglichkeit, die Emulsionsstabilität beim Durchströmen des Gefäßflusses vorherzusagen, eine große Bedeutung. Diese Informationen können gewonnen werden, wenn die Vorstellungen über die Emulsionstruktur klar festgelegt werden. Die Teilchen der Emulsionen haben die Form einer Zweischichtkugel, in deren Mitte PFC (Teilchenkern) und auf deren Oberfläche eine Emulgatorschicht (Hülle) liegt [12]. Die Hüllendicke des Emulgators ist gering und beträgt 5-10 % des Teilchendurchmessers. Das Verhalten der Emulsionen im Blutstrom (Zusammenwirkung mit Plasmaproteinen und Blutzellen, Ausscheidegeschwindigkeit usw.) und die Stabilität bei Dauerlagerung hängen aber von der Festigkeit und dem Zustand der grenzflächenaktiven Substanz um die Teilchen stark ab. Deswegen ist es nötig, gleichzeitig Informationen über die Teilchengröße und die Strukturänderung in den zu untersuchenden Medien bei solchen oder anderen Einwirkungen zu erhalten.

Zur theoretischen Beschreibung und Analyse der Strukturänderung in Emulsionen als Basis für Infusionsmedien sollen folgende Vorstellungen hervorgehoben werden [13]:
1) Die "Gesamtstruktur" der Emulsionen und deren Änderung wird durch den mittleren Teilchendurchschnitt und die Verteilung gemäß der Teilchengröße gekennzeichnet.
2) Die "Mikrostruktur" ist durch den Emulgatorzustand in der Hülle und den Grad der Zusammenwirkung des Emulgators mit PFCs, die gegenseitige Lage der Tensidmoleküle, deren Ordnung, Packungsdichte, Oxidationsgrad, und den Phasenzustand der strukturierten Moleküle gekennzeichnet.

Bis jetzt beschränkten sich alle Forscher auf die Analyse einer "allgemeinen Struktur", die absolut nicht ausreichend ist, da die Emulsionstabilität, Biokompatibilität und insbesondere die Teilchenoberflächeneigenschaften und die Absorptionsfähigkeit der Teilchen durch die Mikrostruktur bestimmt werden.

Die Emulsionen gemäß der Erfindung wurden mit dem Prototyp verglichen und vor allem auf Parmeter untersucht, die die Änderung der allgemeinen Struktur zu unterschiedlicher Lagerungszeit fertiger Emulsionen kennzeichnen.

Zweitens wurde die Einwirkung von zerstörenden Faktoren auf die Emulsion unter Bedingungen simuliert, die den Mikrostrukturzustand der Emulsion bewerten lassen. Es wurde nämlich eine "Stresseinwirkung" in Form einer Verdünnung mit Wasser eingesetzt und eine bestimmte Änderung der Parameter im Vergleich zur nativen Emulsion vorgenommen. Die Wasserverdünnung der Emulsionen stört das eingestellte Gleichgewicht zwischen der Absorptionsschicht der Tenside (Hülle) und den Tensidmolekülen im Dispersionsmedium. Deswegen hat es eine bestimmte, prognostizierende Aussagekraft in Bezug auf die Stabilitätserhaltung des metastabilen Systems (Fluorkohlenstoffemulsion) oder auf dessen Zerfall.

Darüber hinaus wurde die Änderung der Mikrostruktur und die Kompatibilität der Emulsionen beim Kontakt mit Blutserum als Systemmodell (Untersuchung der Biokompatibilität der Emulsion bei Versuchen in vitro) untersucht. Die Zusammenwirkung zweier heterogener disperser Systeme, das Blutserum und die Fluorkohlenstoffemulsion, charakterisiert die Änderung oberflächlicher Teilcheneigenschaften beim Eindringen in den Blutstrom und die Mikrostrukturänderung der Emulsion beim Lagern. Die Änderung der allgemeinen Struktur und der Mikrostruktur wurde in gleichen Zeiträumen im Laufe von 12 Monaten untersucht.

Zur Erkennung der Änderungen der genannten Zustandsparameter beim Lagern wurden Verfahren und Ansätze gebraucht, die zusätzliche Störungen in das zu untersuchende System bei Messungen nicht mitgebracht hätten. Als solche wurden optische Prüfverfahren ausgewählt, geprobt und entwickelt.

Zur Bewertung der allgemeinen Struktur wurde von den Erfindern ein Turbidimetrie-Verfahren oder Trübungsspektrumverfahren [14] gewählt. Dieses Verfahren wurde auch zur Bewertung der Teilchengrößenverteilung in den zu untersuchenden Emulsionen nach dem Zentrifugieren und Fraktionieren eingesetzt. Die Änderung der Mikrostruktur der Emulsion oder der Teilchenoberflächeneigenschaften, die durch Änderung in der Wechselbeziehung der Tensidmoleküle in der Absorptionsschicht um die Fluorkohlenstoffverbindungen herum verursacht wurden, wurden mit einem indirekten Verfahren zur Findung des Wechselwirkungsindex (K_{τ}) der zu untersuchenden Emulsion mit Blutserum in Bezug auf die physiologische Kochsalzlösung bewertet: die relative Trübung K_{τ} = τ₁/τ₂, wobei τ₁ und τ₂ die Trübung der Gemische Serum/Emulsion und Serum/physiologische Kochsalzlösung bei entsprechender Änderung im Verhältnis der Gemischkomponenten bedeuten [15]. Zusätzlich wurden berechnete und experimentelle τ-Werte zur Bestätigung der Naturunveränderlichkeit emulgierter Teilchen gegenübergestellt: τ _{berechnet} = Σ Nᵢ · τᵢ (Σ Nᵢ = 1), wobei τᵢ und Nᵢ die Trübung bzw. den Anteil der ausgeschiedenen Fraktion und τ ₑₓₚₑᵣᵢₘₑₙₜ die Trübung derselben Emulsionsprobe vor dem Fraktionieren bedeuten.

### I. Im Folgenden sind konkrete Zusammensetzungen der Emulsion gemäß der Erfindung angegeben.

### Zusammensetzung 1

Die Emulsion enthält 40 Vol.-% einer Fluorkohlenstoffphase (Cᵥ) aus Perfluordekalin und Perfluoroktylbromid im Verhältnis 1:1 mit einem perfluoriertem Zusatz als Gemisch aus Perfluortripropylamin und seinen Koprodukten: cis- und trans-Isomere von Perfluor-1-propyl-3,4-dimethylpirrolidon und Perfluor-1-propyl-4-methylpiperidin in einer Menge von 50 % vom Gesamtgehalt der Fluorkohlenstoffverbindungen, im emulgierten Zustand mit 5 % Phospholipiddispersion stabilisiert, die Eiphospolipid und Rizinusöl als Adjuvans enthält, dessen Konzentration 15 % vom Summengehalt des Eiphospholipids beträgt, im Wasser-Salz-Medium folgender Zusammensetzung: 2 mmol (115 mg/l) Natriumchlorid, 2mmol einfach substituiertes, phosphorsaueres Kalium (310 mg wasserloses Salz/I), 7,5 mmol zweifach substituiertes, phosphorsaures Natrium (460 mg wasserloses Salz/l), 318 mmol Mannit (58 g Mannitol/l) in Injektionswasser. Der osmotische Druck betrug 310 mosmol/l. Der mittlere durchschnittliche Durchmesser der Emulsionsteilchen betrug 0,195 µm.

### Zusammensetzung 2

Die Emulsion nach der Zusammensetzung 1 zeichnete sich dadurch aus, dass sie 20 Vol.-% einer Fluorkohlenstoffphase (Cᵥ) aus Perfluordekalin und Perfluoroktylbromid im Verhältnis 10:1 mit einem Zusatz als Gemisch aus Perfluortripropylamin und seinen Koprodukten: cis- und trans-Isomere von Perfluor-1-propyl-3,4-dimethylpirrolidon und Perfluor-1-propyl-4-methylpiperidin, mit zusätzlichem Perfluor-N-methylzyklohexylpiperidin in einer Menge von 25 % vom Gesamtgehalt der Fluorkohlenstoffverbindungen, im emulgierten Zustand mit 25 % Phospholipiddispersion stabilisiert, die Sojaphospolipid und Sojaöl als Adjuvans enthält, dessen Konzentration 10 % vom Summengehalt von Eiphospholipid beträgt, im Wasser-Salz-Medium folgender Zusammensetzung: 2mmol einfach substituiertes, phosphorsaures Natrium (276 mg wasserloses Salz/I), 7,5 mmol zweifach substituiertes, phosphorsaures Natrium (460mg wasserloses Salz/I), 278 mmol Mannit (50 g Mannitol/l) in Injektionswasser enthielt. Er osmotische Druck betrug 270 mosmol/l. Der mittlere durchschnittliche Durchmesser der Emulsionsteilchen betrug 0,1 µm.

### Zusammensetzung 3

Die Emulsion nach der Zusammensetzung 1 zeichnete sich dadurch aus, dass sie 15 Vol.-% einer Fluorkohlenstoffphase (Cᵥ) aus Perfluordekalin und Perfluoroktylbromid im Verhältnis 1:10 mit einem Zusatz als Gemisch aus Perfluortripropylamin und seinen Koprodukten: cis- und trans-Isomere von Perfluor-1-propyl-3,4-dimethylpirrolidon und Perfluor-1-propyl-4-methylpiperidin, mit zusätzlichem Perfluor-N-methylzyklohexylpiperidin in einer Menge von 5 % vom Gesamtgehalt der Fluorkohlenstoffverbindungen, im emulgierten Zustand mit 2 % Phospholipiddispersion stabilisiert, die Soja- und Eipospolipid sowie Sonnenblumenkernöl als Adjuvans enthält, dessen Konzentration 5 % vom Summengehalt der Phospholipide beträgt, im Wasser-Salz-Medium folgender Zusammensetzung: 1 mmol einfach substituiertes, phosphorsaures Natrium (138 mg wasserloses Salz/l), 3,7 mmol zweifach substituiertes, phosphorsaures Natrium (230 mg wasserloses Salz/l), 100 mmol Mannit (18 g Mannitol/l) in Injektionswasser enthielt. Der osmotische Druck betrug 105 mosmol/l. Der mittlere durchschnittliche Durchmesser der Emulsionsteilchen betrug 0,08 µm.

### Zusammensetzung 4

Die Emulsion nach Zusammensetzung 1 zeichnete sich dadurch aus, dass sie 10 Vol.-% einer Fluorkohlenstoffphase (Cᵥ) aus Perfluordekalin und Perfluoroktylbromid im Verhältnis 2:1 mit einem Zusatz als Gemisch aus Perfluortripropylamin und seinen Koprodukten: cis- und trans-Isomere von Perfluor-1-propyl-3,4-dimethylpirrolidon und Perfluor-1-propyl-4-methylpiperidin, mit zusätzlichem Perfluor-N-methylzyklohexylpiperidin in einer Menge von 0,2 % vom Gesamtgehalt der Fluorkohlenstoffverbindungen, im emulgierten Zustand mit 2 % Phospholipiddispersion stabilisiert, die Eiphospolipid sowie Sonnenblumen kern- und Sojaöl als Adjuvans enthält, dessen Konzentration 2 % vom Summengehalt der Eiphospholipide beträgt, im Wasser-Salz-Medium folgender Zusammensetzung: 1 mmol einfach substituiertes, phosphorsaures Natrium (138 mg wasserloses Salz/l), 3,7 mmol zweifach substituiertes, phosphorsaures Natrium (230 mg wasserloses Salz/I), 90 mmol Mannit (13 g Mannitol/l) in Injektionswasser enthielt. Der osmotische Druck betrug 100 mosmol/l. Der mittlere durchschnittliche Durchmesser der Emulsionsteilchen betrug 0,07 µm.

### Zusammensetzung 5

Die Emulsion nach Zusammensetzung 1 zeichnete sich dadurch aus, dass sie 2 Vol.-% einer Fluorkohlenstoffphase (Cᵥ) aus Perfluordekalin und Perfluoroktylbromid im Verhältnis 1:2 mit einem Zusatz als Gemisch aus Perfluortripropylamin und seinen Koprodukten: cis- und trans-Isomere von Perfluor-1-propyl-3,4-dimethylpirrolidon und Perfluor-1-propyl-4-methylpiperidin, mit zusätzlichem Perfluor-N-methylzyklohexylpiperidin in einer Menge von 10 % vom Gesamtgehalt der Fluorkohlenstoffverbindungen, im emulgierten Zustand mit 0,2 % Phospholipiddispersion stabilisiert, die Sojaphospolipid sowie Soja- und Rizinusöl als Adjuvans enthält, dessen Konzentration 5 % vom Summengehalt der Sojaphospholipide beträgt, im Wasser-Salz-Medium folgender Zusammensetzung: 2mmol Natriumchlorid (115 mg wasserloses Salz/l), 2 mmol einfach substituiertes, phosphorsaures Natrium (276 mg wasserloses Salz/l), 7,5 mmol zweifach substituiertes, phosphorsaures Natrium (460 mg wasserloses Salz/l), 318 mmol Mannit (58 g Mannitol/l) in Injektionswasser enthielt. Der osmotische Druck betrug 350 mosmol/l. Der mittlere durchschnittliche Durchmesser der Emulsionsteilchen betrug 0,06 µm.

### Zusammensetzung 6

Die Emulsion nach Zusammensetzung 1 zeichnete sich dadurch aus, dass sie 10 Vol.-% einer Fluorkohlenstoffphase (Cᵥ) aus Perfluordekalin und Perfluoroktylbromid im Verhältnis 4:1 mit einem Zusatz als Gemisch aus Perfluortripropylamin und seinen Koprodukten: cis- und trans-Isomere von Perfluor-1-propyl-3,4-dimethylpirrolidon und Perfluor-1-propyl-4-methylpiperidin in einer Menge von 4 % vom Gesamtgehalt der Fluorkohlenstoffverbindungen, im emulgierten Zustand mit 2 % Phospholipiddispersion stabilisiert, die Sojaphospolipid sowie Sonnenblumenkern-, Soja- und Rizinusöl als Adjuvans enthält, dessen Konzentration 4 % vom Summengehalt Sojaphospholipides ist, im Wasser-Salz-Medium folgender Zusammensetzung: 2 mmol einfach substituiertes, phosphorsaures Natrium (276 mg wasserloses Salz/I), 7,5 mmol zweifach substituiertes, phosphorsaures Natrium (460 mg wasserloses Salz/I), 200 mmol Mannit (36 g Mannitol/l) in Injektionswasser enthielt. Der osmotische Druck betrug 225 mosmol/l. Der mittlere durchschnittliche Durchmesser der Emulsionsteilchen betrug 0,09 µm.

In der folgenden Tabelle 6 sind Zusammensetzungen der Emulsionen gemäß der Erfindung nach den Zusammensetzungen 1-6 dargestellt.

**Tabelle 6**

| **Zusammensetzungen 1-6 der Fluorkohlenstoffemulsionen** | | | | | | | |
|---|---|---|---|---|---|---|---|
| Nr. | Cᵥ Vol.-%, PFD/PFOB | Fluorkohlenstoff-Zusatz Relativer Gehalt | Dispersion Phospholipide Gew.-% | Adjuvans Relativer Gehalt | Osmomolarität | Teilchengröße | Wässrige Phase Zusammensetzung in mmol |
| 1 | 40 Vol.-% 1:1 | FI. 50% | Ei-P5% | Rizinusöl 15% | 310 | 0,195 µm | 2 KCl 2 NaH₂P 7,5 Na₂HP 318 Mannit |
| 2 | 20 Vol.-% 10:1 | FI. Perfluormethylzyklohexylpiperidin 25% | Soja-P 2,5% | Sojaöl 10% | 270 | 0,10 µm | 2 NaH₂P 7,5 Na₂HP 278 Mannit |
| 3 | 25 Vol.-% 1:10 | FI. Perfluormethylzyklohexylpiperidin 5% | Ei- und Sojaphospholipid 2% | Sonnenblumenkernöl 10% | 110 | 0,08 µm | 1 NaH₂P 3,7 Na₂HP 100 Mannit |
| 4 | 10 Vol.-% 2:1 | Fl. Perfluormethylzyklohexylpiperidin 0,2% | Eiphospholipid 2% | Sonnenblumenkern- und Rizinusöl 2% | 100 | 0,07 µm | 1 NaH₂P 3,7 Na₂HP 95 Mannit |
| 5 | 2 Vol.-% 1:2 | FI. Perfluormethylzyklohexylpiperidin 10% | Sojaphospholipid 0,2% | Soja- und Rizinusöl 5% | 350 | 0,06 µm | 2 NaCl 2 NaH₂P 7,5 Na₂HP 318 Mannit |
| 6 | 10 Vol.-% 4:1 | Fl. 4% | Sojaphospholipid 2% | Sonnenblumenkern-, Soja- und Rizinusöl 2% | 225 | 0,09 µm | 2 NaH₂P 7,5 Na₂HP 200 Mannit |

### II. Im Folgenden sind konkrete Ausführungsbeispiele zu Herstellungsverfahren der Zusammensetzung der Emulsionen gemäß der Erfindung und die physikalisch-chemische Parameter der Emulsionen angegeben.

### Beispiel 1 Die Emulsion wurde unter aseptischen Bedingungen hergestellt.

1.1. Zur Herstellung wurde 1 I Emulsion mit 10 Vol.-% von PFC zu einer 1 %igen Phospholipiddispersion aufbereitet.
1.2. Erster Schritt Dispersionsaufbereitung: ein steriler, runder Kolben wurde mit 100 ml 10 %iger Alkohollösung von Eiphospholipid befüllt. Der Alkohol wurde in einem Rotationsverdampfer destilliert. 1 g Rizinusöl (Adjuvanskonzentration 10 % vom Eiphospholipidgehalt) und 900 ml Wasser-Salz-Lösung wurden zugegeben.
1.3. Zur Aufbereitung der Wasser-Salz-Lösung wurde apyrogenes Wasser verwendet. Ein Pulver aus einfach substituiertem, phosphorsauerem Natrium, zweifach substituiertem, phosphorsaurem Natrium und kristallines Mannit wurden in einer Trockenkammer bei 110° C 2 Stunden lang getrocknet. Danach wurden 0,138 g wasserloses, einfach substituiertes, phosphoraures Natrium, 0,523 g wasserloses, zweifach substituiertes, phosphoraures Natrium und 50,0 g Mannit in 1 I apyrogenem Wasser in einer Laminareinrichtung unter aseptischen Bedingungen gelöst. Die gewonnene Wasser-Salz-Lösung wurde durch ein steriles Filter der Fa. Millipor mit einer Porengröße von 0,4 µm hindurchgeleitet.
1.4. Ein Gemisch aus pflanzlichem Öl und der Wasser-Salz-Lösung wurde im Kolben mechanisch bis zur Gewinnung einer homogenen Suspension milchiggelblicher Farbe gerührt. Die gewonnene Phospholipidsuspension wurde in einen sterilen Behälter eines Hochdruckhomogenisators gegeben.
1.5. Der Homogenisator wurde vorher mit überhitztem Wasserdampf und 500 ml Alkohol sterilisiert und mit 500 ml apyrogenem Heißwasser gewaschen.
1.6. Die Phospholipidsuspension wurde durch den Homogenisator bei einem Druck von 100 atm bis zur Gewinnung einer halbtransparenten, homogenen Flüssigkeit vierfach hindurchgeleitet. Die gewonnene Dispersion wurde in Glasgefäße abgefüllt. Die Glasgefäße wurden mit sterilem, inerten Gas (Stickstoff, Argon bzw. Gemisch aus Stickstoff und Kohlensäure) 2-4 Minuten lang behandelt.
1.7. Die Glasgefäße wurden mit Gummiverschlüssen verschlossen und mit Alukäppchen gewalzt. Danach wurden die Gefäße bei 100° C 1 Stunde lang hitzesterilisiert. Die Gefäße wurden bei Raumtemperatur bis zum nächsten Herstellungsschritt gelagert.
1.8. Im nächsten Schritt wurden PFCs behandelt. 72 ml PFD wurden mit 8 ml PFOB gemischt. In 80ml dieser Zusammensetzung wurden 20 ml Perfluortripropylamin zugegeben. Die gewonnene Zusammensetzung aus PFD und PFOB mit einem Zusatz von Fluorkohlenstoff wurde mit demselben Volumen medizinischen Alkohols gemischt. Die Perfluorkohlenstoffphase wurde als schwerere mittels eines Trenntrichter vom Alkohol abgetrennt. Das getrennte Gemisch wurde mit einem dreifachen Volumen apyrogenen Wassers gemischt, geschüttelt und im Trenntrichter getrennt (spezifisches Fluorkohlenstoffgewicht überschreitet das spezifische Wassergewicht fast zweifach).
1.9. Danach wurde die Emulsion fertig gestellt. In den Homogenisator wurden 900ml Phospholipiddispersion und 100 ml behandeltes Fluorkohlenstoffgemisch (Zusammensetzung aus PFD/PFOB= 9/1 +PFTPA - 20 %) zugegeben. Der Inhalt wurde mechanisch gerührt und bei einem Druck von 500 atm dispergiert, wobei das ganze Volumen achtfach die Kammer durchfloss, bis zur Gewinnung einer halbtransparenten, gelblichen Flüssigkeit mit Opaleszenzfarbe, der Submikronenemulsion. Die Emulsion wurde in Glasgefäße mit je 100ml eingefüllt. Die Glasgefäße wurden mit Gummiverschlüssen verstopft und mit Alukäppchen gewalzt.
1.10. Die Emulsion in Gefäßen wurde bei 100° C 1 Stunde lang hitzesterilisiert, dann abgekühlt und bei 4° C ein Jahr lang gelagert.

Die gewonnene Emulsion hatte folgende Zusammensetzung: Fluorkohlenstoffphase (Cᵥ) 10 Vol.-%, Verhältnis von PFD zu PFOB 9/1, relativer Gehalt von PFTPA im Fluorkohlenstoffgemisch 20 %, Konzentration von Eiphospholipid 1 Gew.-%, Rizinusölkonzentration 0,1 % (relativer Rizinusölgehalt in Suspension als Adjuvans 10 % vom Gesamtgehalt des Eiphospholipids). Los-Nr.1

Die Viskosität des Loses wurde mittels eines Viskositätsmeters, Modell BΠ 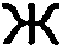 -2, gemessen und betrug 0,953 cP (Zentipoise). Im Vergleich zu Perftoran mit demselben Gehalt an Fluorkohlenstoffphase betrug die Viskosität 2,5 cP (Zentipoise).

### Beispiel 2

Die Emulsion wurde in derselben Zusammensetzung und wie im Beispiel 1 hergestellt. Als Adjuvans wurde ein Gemisch aus Soja- und Rizinusöl im Verhältnis 1:1 ausgewählt. Die Emulsion hatte folgende Zusammensetzung: Cᵥ 10 Vol.-%, PFD/PFOB 9:1, relativer Gehalt an PFTPA 20 %, Eiphospholipidkonzentration 1 Gew.-%, relativer Adjuvansgehalt (Soja-/Rizinusöl 1:1) 10 %. Los-Nr.2.

### Beispiel 3

Die Emulsion wurde wie im Beispiel 1 in derselben Zusammensetzung, aber in einem Volumen von 800ml mit einem Fluorkohlenstoffgehalt von 20 Vol.-% hergestellt. In runden Kolben wurden 200ml 10%-ige Alkohollösung von Sojaphospholipiden zugegeben. Der Alkohol wurde im Rotationsverdampfer destilliert. 3 g Adjuvans (Soja- und Rizinusöl im Verhältnis 1:2, Adjuvanskonzentration 15 % der Eiphospholipide) wurden zugegeben. Die Wasser-Salz-Lösung enthielt 0,276 g wasserloses, einfach substituiertes, phosphorsaures Natrium, 1,046 g wasserloses, zweifach substituiertes, phosphorsaures Natrium und 10,0 g Mannit. In den Kolben mit dem Adjuvans wurde 1 l Wasser-Salz-Lösung zugegeben, geschüttelt und im Homogenisator dispergiert, in Glasgefäße gefüllt und sterilisiert, wie im Beispiel 1. Die Fluorkohlenstoffphase wurde aufbereitet. In 160 ml PFD wurden 40 ml PFOB gegeben. Von dieser Menge wurden 160 ml der Zusammensetzung genommen und mit 40 ml PFTPA gemischt. Nach der Reinigung wurden 200 ml gewonnenes Perfluorkohlenstoffgemisch in den Homogenisator mit 800 ml Dispersion aus Sojaphospholipid tropfweise gegeben. Die gewonnene Emulsion wurde abgefüllt und sterilisiert.

Die Emulsion hatte folgende Zusammensetzung: Cᵥ = 20 Vol.-%, Verhältnis von PFD zu PFOB 8:2, relativer Gehalt von PFTPA 20 %, Sojaphospholipidkonzentration 2 Gew.-%, relativer Adjuvansgehalt (Soja-/Rizinusöl 1:2) 15 %. Los-Nr.3.

### Beispiel 4

Die Emulsion wurde wie im Beispiel 1, jedoch mit einem Verhältnis von PFD zu PFOB von 8:2 hergestellt. Zu 170 ml der Zusammensetzung wurden 30 ml PFMHP gegeben, durch Schütteln gemischt, üblicherweise gereinigt und in einen Homogenisator mit 800 ml Dispersion aus Sojaphospholipid (gewonnen wie im Beispiel 3) und demselben Adjuvans: Soja- und Rizinusöl im Verhältnis 1:2 in einer Menge von 15 % des Sojaphospholüdgehalts tropfweise gegeben. Die Emulsion wurde bei einem Druck von 400 atm dispergiert.

Die Emulsion hatte folgende Zusammensetzung: Cᵥ = 20 Vol.-%, PFD/PFOB-Verhältnis 8:2, relativer Gehalt PFMP 15 %, Sojaphospholipidkonzentration 2 Gew.-%, relativer Adjuvansgehalt (Soja-/Rizinusöl 1:2) 15 %. Los-Nr.4.

### Beispiel 5

Die Emulsion wurde wie im Beispiel 1 nur mit dem Einsatz einer anderen Menge des Eiphospholipids hergestellt. 50 ml Eiphospholipid wurden in runde Kolben gegeben. Der Alkohol wurde im Rotationsverdampfer destilliert. 0,6 g Sonnenblumenkernöl und 0,5 g Salzlösung wurden zugegeben, durch Schütteln gemischt und bei einem Druck von 150 atm homogenisiert. Die Zusammensetzung aus PFD/PFOB im Verhältnis 5:5 wurde durch Mischen mit 25 ml PFD und 25 ml PFOB aufbereitet. 49,5 ml Gemisch wurden mit 0,5 ml PFTPA gemischt. 50ml des Gemisches wurden nach einer Reinigung in einen Homogenisator mit 0,95 I Suspension des Eiphospholipids gegeben. Die Homogenisierung der Vorsuspension wurde unter einem Druck von 350 atm durchgeführt. Das Abfüllen und die Sterilisation der fein verteilten Emulsion wurde nach vorgegebenen Regeln durchgeführt.

Die Emulsion hatte folgende Zusammensetzung: Cᵥ = 5 Vol.-%, PFD/PFOB-Verhältnis 5:5, relativer Gehalt PFTPA 1%, Eiphospholipidkonzentration 0,5 Gew.-%, relativer Adjuvansgehalt Sonnenblumenkernöl, 12 %. Los-Nr.5.

### Beispiel 6

50 ml 10 %iger Alkohollösung von Sojaphospholipid wurden in runde Kolben gegeben. Der Alkohol wurde nach dem vorbeschriebenen Verfahren destilliert. 0,6 g Sojaöl und 950 ml Salzlösung wurden zugegeben. Nach dem Mischen wurde die Dispersion in einem Homogenisator bei einem Druck von 180 atm hergestellt. Nach der Sterilisation wurde die Dispersion zur Fertigstellung der Emulsion verwendet. Eine Zusammensetzung aus PFD und PFOB (im Verhältnis 5:5) wurde durch Mischen mit 25 ml PFD und 25 ml PFOB hergestellt. Zu 49,5 ml dieser Zusammensetzung wurde 0,5 ml PFMHP zugegeben. Nach einer Reinigung mit 50ml Alkohol wurde das Gemisch im Homogenisator mit 950 ml Sojaphospholipiddispersion bearbeitet. Das Homogenisieren wurde in zwei Stufen, wie oben erwähnt, durchgeführt, in der ersten Stufe bei einem Druck von 200atm und in der zweiten Stufe bei einem Druck von 500 atm.

Die gewonnene Emulsion hatte folgende Zusammensetzung: Cᵥ = 5 Vol.-%, PFD/PFOB-Verhältnis 5:5, relativer Gehalt PFMHP 1 %, Sojaphospholipidkonzentration 0,5 Gew.-%, relativer Adjuvansgehalt (Sojaöl) 12 %. Los-Nr.6.

### Beispiel 7

Eine Suspension wurde mit einer Konzentration an Sojaphospholipiden von 0,2 Gew.-% aufbereitet. Dazu wurden 20 ml Alkohollösung des Sojaphospholipids in einen Rotationsverdampfer gegeben. Der Alkohol wurde destilliert. Das Gemisch aus Soja- und Sonnenblumenkernöl im Verhältnis 1:1 als Adjuvans wurde dazugegeben. Das Dispergieren und die Sterilisation mit der Zugabe von 980 ml Salzlösung wurden wie im Beispiel 6 durchgeführt.

Die Zusammensetzung aus PFD/PFOB wurde durch Mischen von 4 ml PFD und 16 ml PFOB (im Verhältnis 2:8) aufbereitet. Zu 19 ml des Gemisches wurde 1ml PFMHP zugegeben. 20 ml des gewonnenen Gemisches aus den drei Komponenten wurde mit 980 ml Suspension homogenisiert. Das Homogenisieren wurde wie im Beispiel oben durchgeführt. Die Sterilisation und das Abfüllen wurden nach Standardverfahren durchgeführt.

Die gewonnene Emulsion hatte folgende Zusammensetzung: Cᵥ = 2 Vol.-%, PFD/PFOB-Verhältnis 2:8, relativer Gehalt PFMHP 5%, Sojaphospholipidkonzentration 0,2 Gew.-%, relativer Adjuvansgehalt (Soja-/Sonnenblumenkernöl 1:1) 1%. Los-Nr.7.

### Beispiel 8

Zur Herstellung der Emulsion wurden 40 Gew.-% Suspension des Eiphospholipids mit einer Konzentration von 5 Gew.-% aufbereitet. Dazu wurden 500ml Alkohollösung des Eiphospholipids in einen Kolben gegeben. Der Alkohol wurde destilliert. 2,5 g Rizinusöl als Adjuvans und 600 ml Salzlösung wurden dazugegeben. Nach dem Mischen wurde das Dispergieren in einem Homogenisator bei einem Druck von 200 atm bis zur Gewinnung eines homogenen Mediums gelblich-weißer Farbe durchgeführt. Die Sterilisation wurde wie oben angegeben durchgeführt.

Die Zusammensetzung wurde durch Mischen mit 4ml PFD und 360 ml PFOB (im Verhältnis 1:9) aufbereitet. Zu 360 ml des Gemisches wurden 40 ml PFMHP zugegeben. 400 ml des gewonnenen Gemisches aus den drei Komponenten wurden mit 600 ml Suspension des Eiphospholipids in zwei Stufen homogenisiert, in der ersten Stufe bei einem Druck von 250 atm und in der zweiten Stufe bei einem Druck von 600 atm. Die Sterilisation und das Abfüllen wurden nach Standardverfahren durchgeführt.

Die Emulsion hatte folgende Zusammensetzung: Cᵥ = 40 Vol.-%, PFD/PFOB-Verhältnis 1:9, relativer Gehalt an PFMHP 10 %, Eiphospholipidkonzentration 5 Gew.-%, relativer Adjuvansgehalt (Rizinusöl) 5 %. Los-Nr.8.

### Beispiel 9

Eine Fluorkohlenstoffphase wurde aus 40 ml PFD und 360 ml PFOB gemischt. Zu 320 ml des Gemisches wurden 80ml zugegeben, und zwar 40 ml PFMHP und 40 ml einer organischen Flüssigkeit. Die Emulgatorsuspension umfasste 4,2 Gew.-% Eiphospholipid, 4,2 Gew.-% Sojaphospholipid und 4,2 g Adjuvans aus Rizinus- und Sonnenblumenkernöl im Verhältnis 9:1, d. h. 5 % des Gesamtgehalts des Eiphospholipids.

Zur Herstellung der Emulsion wurden 600 ml Suspension mit 400 ml des Gemisches aus drei Komponenten in einen Homogenisator gegeben. Das Homogenisieren, Abfüllen und die Sterilisation wurden wie im obigen Beispiel durchgeführt.

Die Emulsion hatte folgende Zusammensetzung: Cᵥ = 40 Vol.-%, PFD/PFOB-Verhältnis 1:9, relativer Gehalt PFMHP und organischer Flüssigkeit 20 %, Phospholipidkonzentration (Ei- und Sojaphospholipid 1:1) 5 Gew.-%, relativer Adjuvansgehalt (Rizinus- und Sonnenblumenkernöl 9:1) 0,25 %. Los-Nr.9.

**Tabelle 7**

| **Zusammensetzung der gewonnenen Emulsionen nach Losen (Beispiele 1-9)** | | | | | | |
|---|---|---|---|---|---|---|
| Los- Nr. | Cᵥ Vol.-%, | Verhältnis PFD/PFOB | Zusatz Relativer Gehalt | Phospholipide Gew.-% | Adjuvans | Adjuvans Relativer Gehalt in % |
| 1 | 10 | 9:1 | FI. 20% | Eiphospholipide 1% | Rizinus | 10 |
| 2 | 10 | 9:1 | Fl. 20% | Eiphospholipide 1% | Rizinus/Soja 1:1 | 10 |
| 3 | 20 | 8:2 | FI. 20% | Sojaphospholipide 2% | Rizinus/Soja 2:1 | 15 |
| 4 | 20 | 8:2 | PFMHP 15% | Sojaphospholipide 2% | Rizinus/Soja 2:1 | 15 |
| 5 | 5 | 5:5 | FI. 1% | Eiphospholipide 0,5% | Sonnenblumenkern | 12 |
| 6 | 5 | 5:5 | PFMHP 1% | Sojaphospholipide 0,5% | Sojaöl | 12 |
| 7 | 2 | 2:8 | PFMHP 5% | Sojaphospholipide 0,2% | Soja/Sonnenblumenkern 1:1 | 1 |
| 8 | 40 | 1:9 | PFMHP 10% | Eiphospholipide 5% | Rizinus/Sonnenblumenkern , 9:1 | 5 |
| 9 | 40 | 1:9 | FI. 10% PFMHP 10% | Eiphospholipide 2,5% Sojaphospholipide 2,5% | Rizinus/Sonnenblumenkern 9:1 | 5 |

In der Tabelle 7 sind Zusammensetzungen aller Lose angegeben. In der Tabelle 8 sind Untersuchungsergebnisse der mittleren Teilchengröße für native (nicht verdünnte) und wasserverdünnte Emulsionen zu unterschiedlichen Lagerzeiten angegeben.

**Tabelle 8**

| **Wellenlängenexponent und mittlere Teilchengröße für native und wasserverdünnte Fluorkohlenstoffemulsionen aus den Beispielen 1, 3, 4, 5, 8 und 9** | | | | | |
|---|---|---|---|---|---|
| Los- Nr. | Lagerung Monate | n | | a, µm | |
| | | nativ | verdünnt 1:2 | nativ | verdünnt 1:2 |
| 1-01 | 0 | 3,40 | 3,20 | 0,114 | 0,13 |
| | 1 | 3,33 | 3,33 | 0,119 | 0,119 |
| | 3 | 3,23 | 3,20 | 0,128 | 0,13 |
| | 6 | 3,27 | 3,23 | 0,124 | 0,128 |
| | 9 | 3,13 | 3, 30 | 0,136 | 0,121 |
| | 12 | 3,05 | 3,14 | 0,143 | 0,135 |
| 1-03 | 0 | 3,27 | 3,33 | 0,125 | 0,119 |
| | 1 | 3,33 | 3,33 | 0,119 | 0,119 |
| | 3 | 3,13 | 3,20 | 0,136 | 0,13 |
| | 6 | 3,20 | 3,27 | 0,130 | 0,124 |
| | 12 | 3,20 | 3,13 | 0,130 | 0,136 |
| 1-04 | 0 | 3,20 | 3,13 | 0,13 | 0,136 |
| | 1 | 3,17 | 3,0 | 0,132 | 0,148 |
| | 3 | 2,87 | 3,10 | 0,165 | 0,14 |
| | 6 | 3,07 | 3,10 | 0,141 | 0,138 |
| | 9 | 3,10 | 3,07 | 0,138 | 0,141 |
| | 12 | 2,94 | 2,85 | 0,155 | 0,17 |
| 1-05 | 0 | 3,20 | 3,27 | 0,13 | 0,124 |
| | 1 | 3,13 | 3,07 | 0,136 | 0,14 |
| | 3 | 3,03 | 3,07 | 0,146 | 0,14 |
| | 6 | 3,07 | 3,03 | 0,141 | 0,145 |
| | 12 | 2,97 | 3,10 | 0,148 | 0,138 |
| 1-08 | 0 | 3,33 | 3,27 | 0,113 | 0,124 |
| | 1 | 3,23 | 3,26 | 0,128 | 0,126 |
| | 3 | 3,10 | 3,20 | 0,139 | 0,13 |
| | 6 | 3,03 | 3,11 | 0,14 | 0,14 |
| | 9 | 2,88 | 3,10 | 0,164 | 0,138 |
| | 12 | 2,87 | 2,9 | 0,165 | 0,160 |
| 1-09 | 0 | 3,16 | 3,20 | 0,134 | 0,13 |
| | 1 | 3,0 | 3,13 | 0,148 | 0,137 |
| | 3 | 3,0 | 3,07 | 0,148 | 0,141 |
| | 6 | 2,86 | 3,02 | 0,182 | 0,157 |
| | 12 | 2,7 | 3,07 | 0,195 | 0,14 |

Der Wert n ist nach dem Verfahren von Kleinstquadraten berechnet worden. Das mittlere Fehlerquadrat in der Bestimmung von n ist 0,01-0,03. Darum ist der Fehler bei der Bestimmung von n = 0,3-1 %. Der Parameter n ist eine charakteristische Funktion des Trübungsspektrumverfahrens und wird nach mindestens 3-5 Punkten gerechnet. Für fein verteilte Emulsionen hängt n eindeutig mit der mittleren Teilchengröße a zusammen [14].

Nach den gewonnenen Ergebnissen ändern sich die durchschnittlichen Parameter n und a bei 12monatiger Lagerung praktisch nicht. Die Wasserverdünnung als Stresseinwirkung beeinflusste die Teilchengröße wenig. Eine geringe Erhöhung der Werte a wurde für Emulsionen mit Sojaphospholipiden zu späteren Zeiten von 9-12 Monaten beobachtet. Der Änderungsbereich für den Wellenlängenexponenten lag für alle Lose der Fluorkohlenstoffemulsionen mit Phospholipiddispersion bei der Lagerung bis zu einem Jahr zwischen 3,4 und 2,7. Dies entsprach der Zunahme der mittleren Teilchengröße von 0,11 bis 0,15-0,195 µm.

Zur Untersuchung der Teilchenverteilung nach der Größe wurde ein Fraktionieren der zu untersuchenden Milieus eingesetzt. Die Emulsionen wurden unter milden Bedingungen (1500 U/min) zentrifugiert und (genau) in 3 Fraktionen getrennt: eine obere Fraktion von 20 %, eine mittlere Fraktion von 60 % und eine untere Fraktion von 20 % des Probenvolumens (Fig. 1). Wie aus Fig. 1 zu erkennen ist, hat die als Prototyp dienende Kohlenstoffemulsion außer den drei nach Teilchengröße unterschiedlichen Fraktionen eine leichte Fraktion, die freie Phospholipide enthält, wodurch schwache Bindungen der Absorptionsschicht mit Ölphase und die in der Absorptionsschicht nicht gebundene Tenside nachgewiesen werden. Für jede Fraktion wurden Werte für a und n gemessen. Die genannten Parameter für fraktionierte Emulsionen der Zusammensetzung gemäß der Erfindung sind bei einer Lagerung von 1-12 Monaten in der Tabelle 9 angegeben. Es stellte sich heraus, dass n und a für obere und mittlere Fraktionen keine Änderung bei der Lagerung aufweisen. Bei der unteren Fraktion wurde eine geringe Erhöhung der Teilchengröße unter einer Vergrößerung der Lagerzeit beobachtet. Dies führte zu einer Erweiterung der Verteilungsbreite der Teilchengröße. Die maximale Verteilungsbreite lag dabei in einem Bereich von 0,06-0,19 µm.

Die gewonnenen Ergebnisse ergaben, dass die mittlere Teilchengröße nativer und wasserverdünnter Emulsionen (Stresseinwirkung) innerhalb von 12 Monaten gering zunahm und in den zulässigen Grenzen unter 0,20 µm blieb.

**Tabelle 9**

| **Parameter n und a, die die Verteilungsbreite der Teilchengröße für Emulsionen aus den Beispielen 1, 3, 4, 5, 8 und 9 bei einer Lagerung von 12 Monaten charakterisieren (obere, mittlere, untere = Fraktionen nach dem Zentrifugieren)** | | | | | | | | |
|---|---|---|---|---|---|---|---|---|
| Los- Nr. | t Monat | Verdünnung | N | | | a µm | | |
| | | | Obere | mittlere | untere | obere | mittlere | untere |
| 1-01 | 0 | unverdünnt | 3,50 | 3,27 | 3,27 | 0,105 | 0,119 | 0,124 |
| | | 1:2 | 3,87 | 3,39 | 2,93 | 0,05 | 0,114 | 0,157 |
| | 1 | unverdünnt | 3,47 | 3,27 | 3,0 | 0,107 | 0,124 | 0,148 |
| | | 1:2 | 3,57 | 3,43 | 3,13 | 0,095 | 0,111 | 0,136 |
| | 3 | unverdünnt | 3,43 | 3,33 | 2,93 | 0,11 | 0,119 | 0,157 |
| | | 1:2 | 3,83 | 3,30 | 3,23 | 0,062 | 0,121 | 0,128 |
| | 6 | unverdünnt | 3,47 | 3,27 | 3,0 | 0,106 | 0,124 | 0,148 |
| | | 1:2 | 3,9 | 3,4 | 3,13 | 0,044 | 0,114 | 0,136 |
| | 12 | unverdünnt | 3,47 | 3,27 | 2,78 | 0,107 | 0,124 | 0,185 |
| | | 1:2 | 3,6 | 3,33 | 2,93 | 0,09 | 0,119 | 0,156 |
| 1-03 | 0 | unverdünnt | 3,37 | 2,87 | 2,83 | 0,118 | 0,165 | 0,175 |
| | | 1:2 | 3,47 | 3,47 | 3,17 | 0,108 | 0,108 | 0,133 |
| | 1 | unverdünnt | 3,4 | 3,08 | 2,8 | 0,114 | 0,141 | 0,183 |
| | | 1:2 | 3,6 | 3,26 | 3,20 | 0,09 | 0,125 | 0,13 |
| | 3 | unverdünnt | 3,33 | 3,23 | 2,87 | 0,119 | 0,128 | 0,165 |
| | | 1:2 | 3,50 | 3,33 | 3,03 | 0,104 | 0,119 | 0,146 |
| | 6 | unverdünnt | 3,53 | 3,33 | 3,10 | 0,101 | 0,119 | 0,139 |
| | | 1:2 | 3,5 | 3,33 | 3,13 | 0,104 | 0,119 | 0,136 |
| | 12 | unverdünnt | 3,53 | 3,27 | 2,93 | 0,10 | 0,124 | 0,157 |
| | | 1:2 | 3,6 | 3,4 | 3,0 | 0,09 | 0,114 | 0,148 |
| 1-04 | 0 | unverdünnt | 3,33 | 3,07 | 3,07 | 0,119 | 0,141 | 0,141 |
| | | 1:2 | 3,3 | 3,17 | 3,13 | 0,121 | 0,131 | 0,136 |
| | 1 | unverdünnt | 3,2 | 3,13 | 2,93 | 0,13 | 0,136 | 0,157 |
| | | 1:2 | 3,33 | 3,23 | 3,03 | 0,119 | 0,128 | 0,145 |
| | 3 | unverdünnt | 3,27 | 3,03 | 2,74 | 0,124 | 0,146 | 0,195 |
| | | 1:2 | 3,3 | 3,2 | 2,8 | 0,121 | 0,13 | 0,182 |
| | 6 | unverdünnt | 3,33 | 3,17 | 2,76 | 0,119 | 0,132 | 0,195 |
| | | 1:2 | 3,50 | 3,20 | 2,93 | 0,104 | 0,13 | 0,157 |
| | 12 | unverdünnt | 3,17 | 3,07 | 2,8 | 0,132 | 0,141 | 0,182 |
| | | 1:2 | 3,33 | 3,20 | 2,93 | 0,119 | 0,13 | 0,157 |
| 1-05 | 0 | unverdünnt | 3,0 | 3,23 | 2,93 | 0,148 | 0,128 | 0,157 |
| | | 1:2 | 3,60 | 3,33 | 3,06 | 0,09 | 0,119 | 0,141 |
| | 1 | unverdünnt | 3,33 | 3,17 | 2,9 | 0,119 | 0,132 | 0,162 |
| | | 1:2 | 3,47 | 3,27 | 3,07 | 0,106 | 0,124 | 0,141 |
| | 3 | unverdünnt | 3,23 | 3,13 | 2,87 | 0,127 | 0,136 | 0,166 |
| | | 1:2 | 3,23 | 3,29 | 3,03 | 0,127 | 0,122 | 0,146 |
| | 6 | unverdünnt | 3,37 | 3,17 | 2,73 | 0,116 | 0,132 | 0,195 |
| | | 1:2 | 3,53 | 3,23 | 2,97 | 0,101 | 0,128 | 0,151 |
| | 12 | unverdünnt | 3,23 | 3,07 | 2,72 | 0,128 | 0,141 | 0,198 |
| | | 1:2 | 3,57 | 3,2 | 2,93 | 0,095 | 0,13 | 0,157 |
| 1-08 | 0 | unverdünnt | 3,40 | 3,37 | 3,27 | 0,114 | 0,116 | 0,124 |
| | | 1:2 | 3,40 | 3,40 | 3,20 | 0,114 | 0,114 | 0,13 |
| | 1 | unverdünnt | 3,37 | 3,30 | 3,17 | 0,116 | 0,122 | 0,132 |
| | | 1:2 | 3,30 | 3,20 | 2,90 | 0,122 | 0,130 | 0,161 |
| | 3 | unverdünnt | 3,33 | 3,17 | 2,93 | 0,119 | 0,132 | 0,158 |
| | | 1:2 | 3,47 | 3,40 | 2,83 | 0,106 | 0,114 | 0,175 |
| | 6 | unverdünnt | 3,20 | 3,06 | 2,81 | 0,13 | 0,151 | 0,181 |
| | | 1:2 | 3,37 | 3,14 | 2,73 | 0,124 | 0,165 | 0,196 |
| | 12 | unverdünnt | 3,08 | 2,97 | 2,72 | 0,14 | 0,15 | 0,198 |
| | | 1:2 | 3,52 | 3,16 | 2,99 | 0,10 | 0,13 | 0,15 |
| 1-09 | 0 | unverdünnt | 3,27 | 3,13 | 2,87 | 0,124 | 0,136 | 0,167 |
| | | 1:2 | 3,27 | 3,30 | 2,83 | 0,125 | 0,122 | 0,175 |
| | 1 | unverdünnt | 3,03 | 3,0 | 2,77 | 0,146 | 0,149 | 0,188 |
| | | 1:2 | 3,30 | 3,03 | 2,93 | 0,122 | 0,139 | 0,157 |
| | 3 | unverdünnt | 3,13 | 3,07 | 2,73 | 0,136 | 0,141 | 0,196 |
| | | 1:2 | 3,17 | 3,10 | 3,0 | 0,132 | 0,138 | 0,148 |
| | 6 | unverdünnt | 3,11 | 3,01 | 2,78 | 0,147 | 0,156 | 0,185 |
| | | 1:2 | 3,40 | 3,17 | 2,34 | 0,124 | 0,136 | 0,162 |
| | 12 | unverdünnt | 3,08 | 2,91 | 2,78 | 0,14 | 0,16 | 0,185 |
| | | 1:2 | 3,33 | 3,15 | 2,81 | 0,12 | 0,135 | 0,18 |

Die beobachtete Vergrößerung mit einer Zunahme der Verteilungsbreite von Teilchengrößen wurde durch Vorfinden in Emulsionen mit relativ großen Teilchen verursacht. Beim Fraktionieren der Emulsionen nach 12monatiger Lagerung nahm die Teilchengröße in der unteren Fraktion von 0,12 bis 0,198 µm zu. Insgesamt stimmen die Ergebnisse mit dem Ostwald-Zerfallmechanismus (bzw. molekulare Destillation) überein. Der Anteil solcher relativ großen Teilchen war so gering (~10 %), dass dies die Zunahme durchschnittlicher Teilchengröße nicht beeinträchtigte. Es soll betont werden, dass beim Fraktionieren der Emulsionen nur eine gleichmäßige Teilchensedimentation beobachtet worden ist, wodurch das Fehlen freier Phospholipide selbst nach einjähriger Lagerung bestätigt wird. Somit blieb die Teilchenverteilung in Emulsionen monomodal. Die gewonnenen Ergebnisse weisen die Beibehaltung einer allgemeinen Struktur der gewonnenen Emulsionen innerhalb von 1-12 Lagermonaten nach.

In der folgenden Tabelle 10 sind Wechselwirkungsindizes Kτ der Teilchen mit Blutserum angegeben, das durch Zugabe einer 5 %igen Albuminlösung im Verhältnis 1:1 1 modifiziert worden ist.

Die Wechselwirkungsindizes der Fluorkohlenstoffemulsionen mit Blutserum, die die Emulsionsmikrostruktur charakterisieren, wiesen einen geringen Schwankungsbereich bei Fluorkohlenstoffemulsionen mit Eiphospholipiden bei 12-monatiger Lagerung auf (Serum-Emulsion-Verhältnis 1:0,05 und 1:0,1). Bei der Vergrößerung des Verhältnisses bis 1:0,10 erweiterte sich auch der Schwankungsbereich von Kτ und Kτ/2. Für Fluorkohlenstoffemulsionen mit Sojaphospholipiden blieb ein geringer Schwankungsbereich für Kτ nur bis zu einer 6-monatigen Lagerung erhalten. Wie erwähnt worden ist, sind die beobachteten Schwankungen eher dadurch begründet, dass es sehr kompliziert ist, das Serumgemisch innerhalb von Versuchsreihen bei unterschiedlicher Lagerungszeit der Emulsionen zu standardisieren. Zugleich weist die Beibehaltung des Schwankungsbereichs für eine Wechselwirkung der Emulsion mit dem Blutserum für jedes Los in bestimmten und engeren Grenzen nach, dass sich die Teilchenoberflächeneigenschaften bei einer Dauerlagerung (6-9 Monate) wenig ändern. Die sprunghafte Änderung von Kτ am Lagerungsende beim Fehlen freier Phospholipide in den Emulsionen kann durch die Entstehung einer zusätzlichen Wechselwirkung zwischen den Teilchen und den Makromolekülen des Serums verursacht werden. Um diese Annahme zu überprüfen, wurden experimentelle Werte und Rechnungswerte der Trübung τ berechnet, die ein zusätzlicher, unabhängiger Parameter zur Bewertung der Strukturganzheit der Fluorkohlenstoffemulsionen ist (Tabelle 11) [13].

**Tabelle 10**

| **Wechselwirkungsindizes der Emulsionen mit dem Blutserum bei unterschiedlicher Lagerungszeit bei einer Temperatur von +4°C** | | | | | |
|---|---|---|---|---|---|
| Los-Nr. | Lagerung | τ Kτ | | | |
| | | Emulsion-Serum-Verhältnis | | | |
| | | 1 : 0,05 | 1 : 0,1 | 1 : 0,05 | 1 : 0,1 |
| 1-02 | 0 | 0,8 ± 0,1 | 1,33 ± 0,03 | 3,8 ± 0,5 | 6,2 ± 0,2 |
| | 1 | 0,93 ± 0,06 | 1,4 ± 0,1 | 4,9 ± 0,3 | 7,2 ± 0,5 |
| | 3 | 0,97 ± 0,02 | 1,99 ± 0,003 | 3,50 ± 0,07 | 5,01 ± 0,02 |
| | 6 | 0,97 ± 0,01 | 1,63 ± 0,03 | 3,5 ± 0,4 | 3,4 ± 0,1 |
| | 9 | 1,23 ± 0,02 | 2,09 ± 0,03 | 3,7 ± 0,2 | 7,0 ± 0,1 |
| | 12 | 1,7 | 2,32 ± 0,01 | 7,2 ± 0,3 | 10,09 ± 0,1 |
| 1-03 | 0 | 0,77 ± 0,07 | 1,1 ± 0,1 | 3,3 ± 0,3 | 4,8 ± 0,4 |
| | 1 | 0,84 ± 0,05 | 1,29 ± 0,06 | 4,0 ± 0,3 | 6,6 ± 0,3 |
| | 3 | 1,0 ± 0,1 1 | 1,46 ± 0,05 | 3,6 ± 0,6 | 5,28 ± 0 |
| | 6 | 0,92 ± 0 | 1,53 ± 0 | 3,3 ± 0,4 | 3,19 ± 0,03 |
| | 12 | 1,33±0 | 1,92±0 | 4,0 ± 0,2 | 6,4 ± 0 |
| 1-04 | 0 | 1,00 ± 0,06 | 1,25 ± 0,08 | 5,4 ± 0,3 | 5,4 ± 0,4 |
| | 1 | 1,1 ± 0,2 | 1,67 ± 0,03 | 5,3 ± 0,9 | 8,9 ± 0,2 |
| | 3 | 1,15 ± 0 | 1,84 ± 0,06 | 4,4 ± 0,3 | 3,83 ± 0,06 |
| | 6 | 1,18 ± 0,02 | 1,97 ± 0,03 | 5,7 ± 0,8 | 10,7 ± 0,2 |
| | 9 | 1,83 ± 0,04 | 1,76 ± 0,02 | 4,2 ± 0,4 | 4,40 ± 0,04 |
| | 12 | 1,30 ± 0,04 | 2,22 ± 0,07 | 7,7 ± 0,5 | 13,9 ± 0,8 |
| 1-05 | 0 | 0,98±0,05 | 1,56±0,08 | 5,1 ± 0,2 | 8,0 ± 0,4 |
| | 1 | 1,1 ±0,1 | 1,76±0,02 | 4,3±0,6 | 7,0±0,1 |
| | 3 | 1,1 ±0,3 | 1,90 ±0,06 | 4,5 ±0,8 | 8,3 ±0,2 |
| | 6 | 1,18 ± 0,02 | 2,05 ± 0,07 | 5,7 ± 0,8 | 11,1 ± 0,4 |
| | 12 | 1,60 ± 0,01 | 2,91 ±0,05 | 3,6 ± 0,3 | 7,2 ± 0,1 |
| 1-08 | 0 | 0,77 ± 0,05 | 1,15±0 | 3,4 ± 0,4 | 5,4 ± 0,7 |
| | 1 | 0,95 ± 0,02 | 1,43 ±0,03 | 4,0 ±1,0 | 6,4 ±0,3 |
| | 3 | 1,1 0 ± 0,1 | 1,57 ± 0,02 | 5 ± 1 | 8,7±0,5 |
| | 6 | 1,4 ±0,1 | 1,94 ±0,03 | 3,3 ±0,6 | 5,4 ± 0,5 |
| | 9 | 1,52 ± 0,03 | 2,68 ± 0,05 | 10 ± 2 | 19 ± 1 |
| | 12 | 1,38 ± 0,08 | 2,30 ± 0,03 | 11 ± 2 | 21,3 ± 0,9 |
| 1-09 | 0 | 0,81 ± 0,02 | 1,48 ± 0,03 | 5,6 ± 0,6 | 14,5 ± 0,3 |
| | 1 | 1,2 ±0,1 | 1,8 ± 0 | 6 ± 1 | 4,79 ± 0,07 |
| | 3 | 1,2 ± 0 | 1,4 ± 0,4 | 6,0 ± 0,5 | 7 ± 3 |
| | 6 | 1,2 ± 0,2 | 2,25 ± 0,03 | 2,8 ± 0,5 | 6,4 ± 0,5 |
| | 9 | 1,9±0,1 | 3,24 ± 0,03 | 14 ± 2 | 24 ± 2 |
| | 12 | 1,52 ± 0,03 | 2,68 ± 0 | 12 ± 1 | 25,3 ± 0,7 |

Physikalisch betrachtet bedeutet τ für disperse Systeme die Summe der Leistungsverluste eines Lichtstrahls bei einigen Teilchen, falls kooperative Wirkungen und mehrfache Dispersionen fehlen. Die Übereinstimmung experimenteller und berechneter τ-Werte für unverdünnte und wasserverdünnte Emulsionen weist nach, dass die Wechselwirkung zwischen Teilchen und Makromolekülen des Serums selbst nach 9-12monatiger Lagerung bei einer Temperatur von +4° C fast unveränderlich bleibt. Die sprunghafte Änderung von Kτ ist eher damit verbunden, dass zusätzliche übermolekulare Strukturen von Perfluorkohlenstoffen/Phospholipiden im wässrigen Dispergens erscheinen, wobei dasselbe Verhältnis von Fluorkohlenstoffen zu Phospholipiden wie bei den Emulsionen auftritt.

**Tabelle 11**

| **Experimentelle Werte und Rechnungswerte der Trübung für Emulsionen mit unterschiedlicher Lagerungszeit** | | | | | |
|---|---|---|---|---|---|
| Los-Nr. PFC Vol.-% | Lagerung Monat | τ₅₀₀ | | | |
| | | unverdünnt | | verdünnt 1:2 | |
| | | Experiment | Rechnung | Experiment | Rechnung |
| 1-02 (10 Vol.-%) | 0 | 9,9 | 10,0 | 4,6 | 4,7 |
| | 1 | 11,7 | 11,6 | 3,60 | 3,75 |
| | 3 | 12,2 | 12,0 | 3,91 | 4,02 |
| | 6 | 13,8 | 13,1 | 4,1 | 3,86 |
| | 9 | 13,8 | 14,0 | 4,29 | 4,37 |
| | 12 | 16,1 | 14,1 | 4,98 | 4,94 |
| 1-03 (20 Vol.-%) | 0 | 8,23 | 9,06 | 3,60 | 3,65 |
| | 1 | 9,66 | 10,2 | 3,07 | 3,11 |
| | 3 | 12,4 | 11,3 | 3,3 | 3,39 |
| | 6 | 13,3 | 11,55 | 3,57 | 3,57 |
| | 12 | 13,1 | 12,4 | 4,02 | 4,02 |
| 1-04 (20 Vol.-%) | 0 | 12,3 | 11,4 | 4,05 | 4,0 |
| | 1 | 13,8 | 13,2 | 4,6 | 4,5 |
| | 3 | 21,0 | 15,4 | 4,98 | 5,0 |
| | 6 | 16,1 | 15,5 | 5,5 | 5,5 |
| | 9 | 17,2 | 17,2 | 5,6 | 6,2 |
| | 12 | 20,9 | 19,9 | 6,8 | 6,5 |
| 1-05 (5 Vol.-%) | 0 | 12,88 | 13,11 | 4,22 | 4,38 |
| | 1 | 14,03 | 13,25 | 4,68 | 4,71 |
| | 3 | 19,1 | 16,9 | 5,6 | 5,56 |
| | 6 | 16,8 | 16,15 | 5,3 | 5,3 |
| | 12 | 18,9 | 18,5 | 5,4 | 4,5 |
| 1-08 (40 Vol.-%) | 0 | 8,97 | 8,74 | 2,91 | 2,81 |
| | 1 | 11,7 | 11,6 | 3,53 | 3,47 |
| | 3 | 13,8 | 13,6 | 4,14 | 4,0 |
| | 6 | 16,6 | 15,4 | 4,68 | 4,59 |
| | 9 | 21,8 | 18,9 | 5,6 | 5,6 |
| | 12 | 21,2 | 19,7 | 6,2 | 6,2 |
| 1-09 (40 Vol.-%) | 0 | 14,03 | 14,44 | 4,29 | 4,15 |
| | 1 | 19,3 | 17,5 | 5,75 | 5,61 |
| | 3 | 19,09 | 18,72 | 5,52 | 5,34 |
| | 6 | 21,8 | 18,2 | 6,13 | 8,71 |
| | 9 | 26,2 | 23,9 | 7,7 | 7,8 |
| | 12 | 26,9 | 25,5 | 4,91 | 4,3 |

Bevor etwas über die Vorteile der Zusammensetzung und des Herstellungsverfahrens gemäß der Erfindung der Fluorkohlenstoffemulsionen gesagt wird, soll unterstrichen werden, dass die Hauptbedingungen zur Erfüllung der Gastransportfunktion der Emulsionen beim Strömen im Blutfluss die Beibehaltung der Korpuskularnatur der Teilchen und keine Reaktogenität sind. Aus der Sicht der Kolloidchemie und Biophysik kann das Gelangen der Emulsion in den Blutstrom als Stresseinwirkung betrachtet werden, die zu einer Änderung der Dispergenseigenschaften führen soll. Diese Einwirkung kann zu folgenden Betrachtungen führen, nämlich zu einer Verdünnung der Emulsion und zu einer Konzentrationsminderung des freien Emulgators im Dispergens (schnelle Phase). Als Ergebnis dieses Prozesses erfolgt eine Schwächung der Molekülbindungen der Tenside mit der Teilchenoberfläche (langsame Phase). Diese Bindungsschwächung der Tenside mit Fluorkohlenstoffen wird noch durch den Kontakt und die Wechselwirkung der Teilchen mit Plasmamakromolekülen beeinträchtigt, wodurch die Zusammensetzung der Absorptionsschicht oder die Teilchenzerstörung geändert werden kann. Die beschriebene Reihenfolge der Prozesse ist eine vereinfachte Darstellung.

In diesseitigen Versuchen simuliert die Verdünnung der Emulsionen mit Wasser die erste Phase, nämlich eine Verdünnung der Emulsion und eine Konzentrationsverringerung des freien Emulgators um die Teilchen. Eine Untersuchung der Wechselwirkung der gewonnenen Emulsionen mit Blutplasma simuliert die zweite Phase, nämlich eine Einwirkung des Kontakts von Serummakromolekülen auf die Eigenschaften der Teilchenoberfläche. Es stellte sich heraus, dass selbst nach einer Lagerung innerhalb eines Jahres die Emulsionsteilchen ihre Mikrostruktur behalten, wobei die Verdünnung mit Wasser die Teilchengröße nicht beeinflusste, wodurch die feste Bindung der Absorptionsschicht der Tenside mit dem Teilchenkern, den Perfluorcarbonen, bestätigt wurde. Der Wechselwirkungsindex der Emulsionsteilchen mit Blutserum (in Grenzen der Meßfehler) blieb auch unverändert, wodurch die Beibehaltung der Teilchenoberflächeneigenschaften nachgewiesen wurde. Die Rechnungswerte τ (nach dem Fraktionieren) stimmten mit den experimentellen Werten (vor dem Fraktionieren) überein, wodurch die Beibehaltung der Teilchennatur (Teilchenstruktur) und das Fehlen freier Phospholipide in den Emulsionen nach einem Jahr Lagerung bestätigt wurden.

Die eingesetzten, methodischen Ansätze erhöhen deutlich die Richtigkeit der Vorhersage über die Stabilität der Emulsionen beim Gelangen in den Blutstrom. Als Nachweis des Gesagten dienen Ergebnisse paralleler Untersuchungen der Stabilität und die Bestimmung des Reaktogenitätsindexes (Ip) mehrer Lose identischer Zusammensetzung. Der Index Ip ist nach der Methode [3] bestimmt worden.

### Beispiel 10

Vier Lose gleicher Emulsionen mit einer Zusammensetzung nach Beispiel 2 wurden hergestellt: Fluorkohlenstoffbasis 9±1 Vol.-%, Verhältnis PFD zu PFOB 9:1, Zusatz einer organischen Flüssigkeit 20 %, Eiphospholipide 1 Gew.-%, Adjuvans (Rizinus- und Sojaöl 1:1) 8 % der Eiphospholipidkonzentration.

In der folgenden Tabelle 12 sind Werte für n und a dieser Lose mit unterschiedlichen Lagerungszeiten dargestellt.

**Tabelle 12**

| **Wellenlängenexponent n und durchschnittliche Teilchengröße a für Emulsionen gleicher Zusammensetzung PFD/PFOB/ FI/Ei-P mit unterschiedlichen Lagerungszeiten** | | | | | |
|---|---|---|---|---|---|
| Los-Nr. | Lagerung (Monate) | n | a µm | n | a µm |
| | | unverdünnt | | verdünnt | |
| 5-03 | 0 | 3,70 ± 0,03 | 0,08 | 3,81 ± 0,07 | 0,06(5) |
| | 1 | 3,62 ± 0,03 | 0,09 | 3,83 ± 0,05 | 0,06(5) |
| | 6 | 3,80 ± 0,04 | 0,07 | 3,76 ± 0,06 | 0,07(5) |
| 5-04 | 0 | 3,36 ± 0,02 | 0,117 | 3,58 ± 0,01 | 0,09 |
| | 1 | 3,30 ± 0,04 | 0,122 | 3,53 ± 0,05 | 0,10(1) |
| | 6 | 3,37 ± 0,05 | 0,117 | 3,08 ± 0,08 | 0,14 |
| 5-05 | 0 | 3,35 ± 0,05 | 0,116 | 3,47 ± 0,04 | 0,11 |
| | 1 | 3,07 ± 0,07 | 0,141 | 3,24 ± 0,09 | 0,12(7) |
| | 6 | 3,37 ± 0,03 | 0,117 | 3,13 ± 0,02 | 0,13(6) |
| 5-06 | 0 | 3,32 ± 0,02 | 0,12 | 3,35 ± 0,05 | 0,11(6) |
| | 1 | 3,07 ± 0,07 | 0,141 | 3,09 ± 0,08 | 0,139 |
| | 6 | 3,45 ± 0,04 | 0,108 | 3,26 ± 0,09 | 0,12(5) |

**Tabelle 13**

| **Wellenlängenexponent n und mittlere Teilchengröße a, die die Verteilungsbreite der Teilchen nach der Größe charakterisieren, beim Fraktionieren mittels Zentrifugieren der Emulsionen mit derselben Zusammensetzung PFD/PFOB/ FI/Eiphospholipid** | | | | | | | | |
|---|---|---|---|---|---|---|---|---|
| Los- Nr. | Lagerung (Monate) | Wasserverdünnung | n | | | a µm | | |
| | | | obere | mittlere | untere | obere | mittlere | untere |
| 5-04 | 0 | Nativ | 3,53 | 3,47 | 3,21 | 0,10 | 0,11 | 0,13 |
| | | 1:2 | 3,77 | 3,63 | 3,27 | 0,07 | 0,09 | 0,124 |
| | 1 | Nativ | 3,53 | 3,51 | 3,19 | 0,101 | 0,104 | 0,132 |
| | | 1:2 | 3,85 | 3,66 | 3,26 | 0,06, | 0,085 | 0,125 |
| | 6 | Nativ | 3,61 | 3,46 | 3,15 | 0,089 | 0,109 | 0,135 |
| | | 1:2 | 3,61 | 3,12 | 2,9 | 0,089 | 0,136 | 0,160 |
| 5-05 | 0 | Nativ | 3,71 | 3,43 | 3,17 | 0,079 | 0,110 | 0,132 |
| | | 1:2 | 3,88 | 3,67 | 3,31 | 0,055 | 0,084 | 0,120 |
| | 1 | Nativ | 3,60 | 3,30 | 3,05 | 0,094 | 0,122 | 0,145 |
| | | 1:2 | 3,72 | 3,35 | 2,78 | 0,078 | 0,122 | 0,184 |
| | 6 | Nativ | 3,56 | 3,34 | 2,90 | 0,098 | 0,118 | 0,162 |
| | | 1:2 | 3,53 | 3,15 | 2,71 | 0,101 | 0,135 | 0,20 |

Nach den gewonnenen Daten blieb der mittlere Teilchendurchschnitt in allen Fällen für native und wasserverdünnte Emulsionen in der 6monatigen Lagerzeit unverändert, wobei er im Bereich von 0,06-0,17 µm lag. Die Verteilungsbreite der Teilchengröße für native und wasserverdünnte Emulsionen der angegebenen Zusammensetzung änderte sich in der angegebenen Untersuchungszeit (Tabelle 13) praktisch nicht. Der Wechselwirkungsindex Kτ der gewonnenen Emulsionen mit modifiziertem Blutserum in Bezug auf den relativen Messfehler (± 10 %) schwankte in engeren Grenzen (s. folgende Tabelle 14).

**Tabelle 14**

| **Wechselwirkungsindex Kτ der Emulsionen gleicher Zusammensetzung PFDIPFOB/ FIIEiphospholipid mit dem mit Albumin (80 %) modifizierten Blutserum** | | | |
|---|---|---|---|
| Los-Nr. | Lagerung (Monate) | Verhältnis von Serum zu Emulsion | |
| | | 1:0,05 | 1:0,1 |
| 5-03 | 0 | 2,28 | 3,37 |
| | 1 | 2,34 | 3,70 |
| | 6 | 2,60 | 4,00 |
| 5-04 | 0 | 3,76 | 6,00 |
| | 1 | 3,63 | 5,62 |
| | 6 | 4,05 | 6,03 |
| 5-05 | 0 | 4,0 | 5,56 |
| | 1 | 4,33 | 6,06 |
| | 6 | 4,53 | 6,1 |

**Tabelle 15**

| **Reaktogenitätsindex der Emulsionen gleicher Zusammensetzung PFD/PFOB/ FI/Eiphospholipid (zur Prüfung wurde eine Dispersion des Eiphospholipids eingesetzt)** | | | |
|---|---|---|---|
| Los-Nr. | Lagerzeit (Monate) | | |
| | 0 | 1 | 6 |
| Eiphospholipiddispersion | 1,4 | -- | -- |
| 5-03 | 2,83 | 1,68 | 1,92 |
| 5-04 | 1,14 | 2,14 | 2,24 |
| 5-05 | 1,83 | 1,83 | 1,70 |
| 5-06 | 2,42 | 1,35 | 2,63 |

Die eingeführten Ergebnisse sprechen dafür, dass die Emulsionen und das Herstellungsverfahren gemäß der Erfindung eine hohe Qualität der Emulsionsmikrostruktur zulassen, ohne dass diese bei einer Lagerung im nicht eingefrorenen Zustand und bei einer nachfolgenden Stresseinwirkung in vitro (Wasserverdünnung, Wechselwirkung mit dem mit Albumin angereicherten Blutserum) beeinträchtigt wird. Die Ergebnisse der Reaktogenitätsprüfung derselben Emulsionsmuster bestätigen die Ergebnisse der Simulationsuntersuchungen vollständig. Zu keinem Zeitpunkt der Untersuchung überschritt der Reaktogenitätsindex den kritischen Wert 3 (s. Tabelle 15).

### Beispiel 11

Die Unversehrtheit der Struktur und die Untersuchung der Reaktogenität von Emulsionen mit einem niedrigen Fluorkarbongehalt von 5 Vol.-%.

Die Emulsion hatte folgende Zusammensetzung: PFD/PFOB 1:1, PFMHP 1 %, Sojaphospholipid 0,5 %, Sojaöl als Adjuvans 12 %, Verteilungsbreite der Teilchengröße im Bereich von 0,03 bis 0,12 µm, Anfangssreaktogenität 1,61. Untersucht wurden die Änderungen der mittleren Teilchengröße von nativen und wasserverdünnten, gleichen Emulsionen vierer Lose (s. Tabelle 16) und die Reaktogenität nach 6 Lagermonaten (s. Tabelle 17). Wie aus den angegebenen Daten erkennbar ist, sichern die Zunahme der Teilchengröße bei der eingesetzten Rezeptur und dem Herstellungsverfahren gemäß der Erfindung die Gewinnung einer niedrigen Reaktogenität.

**Tabelle 16**

| **Wellenlängenexponent n und mittlere Teilchengröße a nativer und wasserverdünnter Emulsionen gleicher Zusammensetzung PFD/PFOB/ FI/Sojaphospholipid** | | | | | |
|---|---|---|---|---|---|
| Los-Nr. | Lagerung (Monate) | n | a µm | n | a µm |
| | | unverdünnt | | wasserverdünnt 1:2 | |
| 6-02 | 1 | 3,27 ± 0,04 | 0,122 | 3,29 ± 0,03 | 0,123 |
| | 6 | 3,08 ±0,09 | 0,138 | 3,18 ± 0,02 | 0,132 |
| 6-03 | 1 | 3,19 ±0,01 | 0,13 | 3,28 ± 0,02 | 0,124 |
| | 6 | 3,06 ± 0,01 | 0,145 | 3,11 ±0,03 | 0,138 |
| 6-05 | 1 | 3,31 ± 0,01 | 0,12 | 3,48 ± 0,01 | 0,105 |
| | 6 | 3,24 ±0,01 | 0,126 | 3,39 ± 0,01 | 0,114 |
| 6-06 | 1 | 3,11 ±0,03 | 0,137 | 3,23 ± 0,03 | 0,128 |

**Tabelle 17**

| **Reaktogenität Ip für Emulsionen mit niedrigem Fluorkarbongehalt nach 6 Lagermonaten im nicht eingefrorenen Zustand** | | | | |
|---|---|---|---|---|
| Los-Nr. | 6-02 | 6-03 | 6-05 | 6-06 |
| Reaktogenität | 1,87 | 2,00 | 1,36 | 1,8 |

### Beispiel 12

Die Dauerlagerung der Emulsion innerhalb von 18 Monaten, die 10 Vol.-% an Fluorkohlenstoffen enthält, ein Verhältnis PFD/PFOB 8:2, eine organische Flüssigkeit von 20 %, ein Eiphospholipid von 2 % und Rizinusöl als Adjuvans von 10 % aufweist. Die Untersuchungsergebnisse mittlerer Teilchengröße bei verschiedenen Lagerungszeiten und Wasserverdünnungen sind in der Tabelle 18 angegeben. Die Entwicklung der Wechselwirkung der Emulsion mit dem bis 50 % mit Albumin angereicherten Blutserum sind in der Tabelle 19 angegeben.

**Tabelle 18**

| **Wellenlängenexponent n und durchschnittliche Teilchengröße a für native und wasserverdünnte Emulsionen PFDIPFOB/ Fl/Ei-P mit unterschiedlicher Lagerzeit im nicht eingefrorenen Zustand** | | | | | |
|---|---|---|---|---|---|
| Los-Nr. | Lagerung (Monate) | N | a µm | n | a µm |
| | | unverdünnt | | wasserverdünnt 1:2 | |
| 7-03 | 0 | 3,70 ± 0,03 | 0,08 | 3,81 ± 0,07 | 0,065 |
| | 1 | 3,62 ± 0,03 | 0,09 | 3,83 ± 0,05 | 0,065 |
| | 6 | 3,80 ± 0,04 | 0,07 | 3,76 ± 0,06 | 0,07 |
| | 18 | 3,81 ± 0,04 | 0,065 | 3,77 ± 0,06 | 0,07 |

**Tabelle 19**

| **Wechselwirkung Kτ der Emulsion PFD/PFOB/ FI/Ei-P mit dem mit Albumin (50 %) modifizierten Blutserum bei unterschiedlichen Lagerzeiten** | | | |
|---|---|---|---|
| Los-Nr. | Lagerung (Monate) | Kτ | |
| | | Verhältnis von Serum zu Emulsion | |
| | | 1 :0,05 | 1:0,1 |
| 7-03 | 0 | 1,74 | 2,37 |
| | 1 | 1,68 | 2,25 |
| | 6 | 1,48 | 2,74 |
| | 18 | 1,22 | 1,78 |

Wie aus den obigen Daten hervorgeht, behält die gewonnene Emulsion messbare, physikalisch-chemische Eigenschaften bei. Aufgrund dieser Tatsache beträgt die Reaktogenität der Emulsion nach 18 Lagermonaten 1,5.

### Beispiel 13

Prototypen, Oxygent AF 0104 (Hersteller Alliance Therapeutic, USA), und die nach dem Verfahren gemäß der Erfindung hergestellte Emulsion wurden hinsichtlich ihrer Qualität gegenübergestellt. Der Vergleich wurde abhängig von der Änderung des Wellenlängenexponenten und der mittleren Teilchengröße unter Wasserverdünnung durchgeführt.

In den zu vergleichenden Emulsionen mit unterschiedlichem, absoluten Fluorkohlenstoffgehalt wurde ein gleiches Verhältnis von Fluorkarbonen zu Phospholipiden eingehalten. Die angegebenen Emulsionen unterscheiden sich nach dem Herstellungsverfahren. Als Folge enthält die Emulsion PFOB-2 (hergestellt nach dem Verfahren gemäß der Erfindung) keine freie Phospholipidphase (s. Fig. 16) nach dem Zentrifugieren, wobei der Oxygent und der Prototyp PFO-1 in der Absorptionsschicht nicht gebundene und freie Phospholipide umfassen, die bei dem Zentrifugieren leicht aufschwimmen (s. Fig. 1A). Darum verkleinern sich die mittlere Teilchengröße beim Oxygent auf 0,35 bis 0,15 bei einer Verdünnung der Emulsion mit Wasser, wobei die Phospholipidaggregate und die Emulsionsteilchen zerfallen.

In der Prototyp-Emulsion (PFOB-1) fehlten offensichtlich solche groben Aggregate. Ihr Vorhandensein aber, abgesehen von den Zentrifugierungsergebnissen, weist starke Unterschiede zwischen berechneten und experimentellen Trübungsparametern auf, die nach der Additivregel für native und verdünnte Emulsionen bestimmt werden. Für die Emulsionen nach dem Herstellungsverfahren gemäß der Erfindung wurde praktisch eine volle Übereinstimmung der experimentelle und berechneten Trübungswerte beobachtet (s. Tabelle 21). Es soll bemerkt werden, dass aus physikalischer Sicht der zu bestimmende Parameter die Summe der Leistungsverluste eines Lichtstrahls in einzelnen Teilchen darstellt, falls kooperative Wirkungen und eine mehrfache Streuung fehlen. Keine Übereinstimmung der experimentellen und berechneten Trübungswerte für den Oxygent und den Prototyp bestätigt die Nichterfüllung der Additivregel, d. h. das eine zusätzliche Wechselwirkung zwischen den Teilchen und dem Lichtstrom in den genannten, dispersen Systemen auftritt. Diese Wechselwirkung ist bei der Wasserverdünnung des Oxygents und Prototyps klar erkennbar, wodurch keine Homogenität der Teilchenarten erreicht wird, sondern unterschiedliche Mizellarstrukturen von Phospholiiden neben den Teilchen der Fluorkohlenstoffemulsionen erreicht werden. Für die Emulsionen PFOB-2 und PFD/PFOB (Los-Nr. 5-03) erfüllte die Wechselwirkung der Teilchen mit Lichtstrom die Additivregel selbst nach einem Lagermonat im nicht eingefrorenen Zustand. 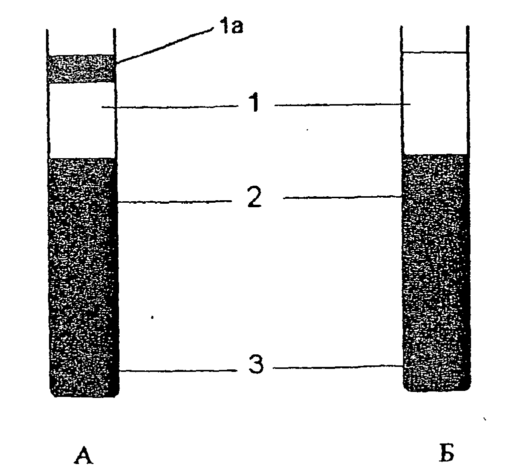 Fig. 1: Trennung der Fluorkohlenstoffemulsionen in Fraktionen abhängig vom Herstellungsverfahren: A nach dem Prototyp (die Emulsion enthält freie Phospholipide), B nach dem Verfahren gemäß der Erfindung (die Emulsion enthält nur Teilchen unterschiedlicher Größe). 1, 2, 3 bedeuten die obere, mittlere und untere Fraktion. 1a bedeutet die freien Phospholipide in der oberen Fraktion.

**Tabelle 20**

| **Zusammensetzung unterschiedlicher Fluorkohlenstoffemulsionen, Wellenlängenexponent n und mittlere Teilchengröße a für unverdünnte und wasserverdünnte (1:1) Emulsionen** | | | | | | |
|---|---|---|---|---|---|---|
| Präparat, PFC | Zusammensetzung | | n | | a, µm | |
| | PFC Gew./Vol. | Phospholipid Gew.-% | unverdünnt | Wasserverdünnung H₂O 1:1 | unverdünnt | Wasserverdünnung H₂O 1:1 |
| Oxygent AF 0104 | 90 Gew.-% | 4 | 2,34 ± 0,04 | 2,93 ± 0,1 | 0,35 | 0,15 |
| (PFOB) | 45 Vol.-% | | | | | |
| Emulsion PFOB-1 | 45 Gew.-% | 2 | 2,91 ± 0,09 | 2,82 ± 0,10 | 0,16 | 0,18 |
| (Prototyp) | 22 Vol.-% | | | | | |
| Emulsion nach erfindungsgemäße m Verfahren (Zusammensetzung nach Prototyp PFOB-2) | 20% 10 Vol.-% | 1 | 3,38 ± 0,02 | 3,33 ± 0,05 | 0,114 | 0,115 |

**Tabelle 21**

| **Übereinstimmung experimenteller und berechneter Trübungswerte nach der Additivregel** | | | | |
|---|---|---|---|---|
| Präparat | unverdünnt | | wasserverdünnt 1:1 | |
| | Versuch | Berechnung | Versuch | Berechnung |
| Oxygent | 133,9 ±1,0 | 33,2 | 59,3 ± 0,5 | 23 |
| PFOB-1 (Prototyp) | 26,6 ± 0,3 | 18,6 | 16,4 ± 0,2 | 5,21 |
| PFOB-2 (Verfahren gemäß der Erfindung) | 9,3 | 10,0 | 2,35 | 2,31 |
| PFD/PFOB 9:1 (Zusammensetzung und Verfahren gemäß der Erfindung, Beispiel 10) | 12,9 | 11,2 | 3,24 | 3,17 |

Somit beweisen die angegebenen Beispiele eine ganze Reihe von Vorteilen der beschriebenen Zusammensetzung und des beschriebenen Herstellungsverfahrens der Emulsion gemäß der Erfindung im Vergleich zu den Prototypen und der der Erfindung am nächsten kommenden Emulsionen. Dies ist aufgrund der nachfolgenden Synergieeffekten möglich.
1. PFD und PFOB sind als Hauptkomponenten ausgewählt, da sich diese Perfluorkohlenstoffe nach ihren biologischen und physikalisch-chemischen Eigenschaften als biologisch akzeptierbar erweisen und eine bewährte und schnelle Ausscheidegeschwindigkeit aus dem Organismus, d. h. aus den Zellen des Retikulendothelsystems, aufweisen, die Fluorkohlenstoffteilchen akkumulieren.
2. Die gemeinsame Verwendung von PFD und PFOB im wirksamen Verhältnis führt zu einer gemischten Ölphase, deren Eigenschaften sich von der Mitte zur Peripherie graduell ändern. Diese lässt zu, in der Rezeptur wenig lipophile und keine hydrophile, tertiäre Amine einzusetzen, die einen wesentlich niedrigeren Dampfdruck aufweisen (s. Tabelle 1) und somit die Diffussion lipophiler Moleküle von PFD und PFOB in die wässrige Phase vermindern. Dies verlangsamt die Geschwindigkeit des Hauptmechanismus des Emulsionszerfalls, die Ostwald'sche Reifung, und erhöht die Stabilität der ausgewählten Zusammensetzung der Fluorkohlenstoffemulsionen.
3. Die Einführung von PFOB in die Zusammensetzung der Emulsionen erweitert deren Sauerstoffaufnahmefähigkeit bei demselben Fluorkohlenstoffgehalt und bringt zusätzliche Röntgenkontrasteigenschaften mit.
4. PFOB/PFD und ein Gemisch aus tertiären Aminen verhilft zu einer niedrigen Viskosität der Endform aufgrund der stärkeren Bindung der Absorptionsschicht der Tenside um die Teilchen, wodurch ermöglicht wird, freie, mizellare Formen von Phospholipiden in der wässrigen Phase der Emulsionen auszuschließen.
5. Der Einsatz von Ölen unterschiedlicher physikalisch-chemischer Eigenschaften neben den Phospholipiden fördert die Bildung einer dichteren membranähnlichen Absorptionsschicht um die Teilchen mit einer geringeren Menge an Phospholipide und verhindert mizellare Strukturen ohne Fluorkohlenstoffe.
6. Die Besonderheiten des eingesetzten Wasser-Salz-Milieus sichert eine negative Ladung auf der Teilchenoberfläche, wodurch die Koaleszenz der Teilchen bei der Lagerung und beim Transport verhindert wird.
7. Neben verfahrenstechnischen Methoden, die die Herstellung einer hochkalibrierten (mit enger Teilchenverteilung) Emulsion sichern, schwächen die o. g. Methoden die Molekulardestillation und fördern eine höhere Stabilität der Emulsionen.
8. Die fehlenden Aggregate der Teilchen und mizellaren Formen der Phospholipide sichern die Absorptionseigenschaft und die das Komplement aktivierende Eigenschaft der Emulsion im Blutstrom, wodurch eine niedrige Reaktogenität bewirkt und eine erhöhte Biokompatibilität der Emulsionen der Zusammensetzung gemäß der Erfindung gefördert wird.

### III. Nachstehend sind Versuche für den biomedizinischen Einsatz der Emulsionen gemäß der Erfindung angegeben.

### Versuch 1

Für den Einsatz der Emulsionen für einen massiven Blutersatz wurde bei gesunden Ratten Wistar mit einem Gewicht von 250-300 g (n=20) eine Volumensubstitution mit einer Fluorkohlenstoffemulsion, hergestellt nach Beispiel 1 (Abschnitt II), unter einer Nembutal-Narkose durchgeführt. Die Überlebungsfähigkeit nach massivem Blutersatz und unter Gewinnung von Lebermitochondrien nach einem Ausgleich des Blutverlustes (s. Verfahren [16]) wurde bestimmt. Zur Sicherstellung des onkotischen Drucks nach dem massiven Blutersatz wurde die Emulsion vor der Infusion mit einer 20 %igen, menschliches Albumin im Verhältnis von 1 Teil Albumin zu 6 Teilen Emulsion gemischt, so dass eine Endkonzentration an Albumin von 3,5 % erreicht wurde (bezogen darauf, dass die Emulsion 10 Vol.-% an Fluorkohlenstoffen aufwies). Während des Blutersatzes atmeten die Ratten eine mit Sauerstoff bis auf FiO₂ = 0,5 angereicherte Luft ein, die unter einer transparenten Plexiglashaube zugeführt worden war. Die Haube deckte den Kopf des auf dem Rücken fixierten Tiers ab. Aus dem Venensinus (des rechten Vorhofs) wurden 3,5 ml Blut entnommen, und 3,5 ml Emulsion wurden in den Venensinus injiziert. Nach 10 Minuten wurden 3,5 ml Blut entnommen und dieselbe Menge Emulsion injiziert. Diese Vorgehensweise wurde wiederholt, bis die entnommene Blutmenge durchschnittlich mindestens 3,5 % des Gewichts des Tiers umfasste; beispielsweise betrugen für das Gewicht von 250 g die entnommene Blutmenge und die injizierte Emulsion je 8,8 ml. Vor und nach dem Blutersatz wurden das Hämoglobin im Peripherieblut, der Partialdruck von Sauerstoff und der pH-Wert im arteriellen und Venenblut bestimmt. In dieser Versuchsreihe sank die Hämoglobinkonzentration nach dem Blutersatz durchschnittlich um 1,9. In der Kontrollgruppe (n=20) wurde eine Lösung von 0,15 mol Natriumchlorid und 3,5 %igem Albumin statt der Emulsion injiziert. Mit dem Kernresonanz-Spektrometer wurde der Fluorkohlenstoffgehalt im Peripherieblut bestimmt. Nach dem Blutersatz wurden die Tiere in einer Kammer gehalten, der eine mit Sauerstoff bis FiO₂ = 0,5 angereicherte Luft zugeführt wurde.

In der Versuchsgruppe (mit Blutersatz) überlebten alle Tiere und die Hämoglobin-, Erythrozyten- und Leukozytenwerte wurden innerhalb von 5 Tagen auf die Normwerte gebracht. In der Kontrollgruppe starben 3 Tiere. Nach 5 Tagen wurden alle Tiere unter einer Nembutal-Narkose getötet, und aus der Leber wurden Mitochondrien getrennt. Das Atmen der Lebermitochondrien wurde polarimetrisch in der geschlossenen, thermostatiesierenden Zelle bei 27° C registriert. Es wurde festgestellt, dass die Atmungsgeschwindigkeit im aktiven Zustand (mit ATP-Synthese) und die ATP-Synthesegeschwindigkeit mit Oxidation des NADabhängigen Substrats von 3-Hydrooxybutyrat durchschnittlich um 1,5 bei einer 20 %igen Aktivierung der Sukzinatoxidation abnehmen. Diese Daten weisen eine mäßige Ischämieverletzung der Leber nach. In den nach 5 Tagen seit dem Blutersatz getrennten Lebermitochondrien wurde eine Aktivierung aller Atmungsgeschwindigkeiten und eine ATP-Synthese von durchschnittlich 25 % beobachtet, wodurch eine mäßige Hypoxie in der Anamnese bewiesen wurde.

### Versuch 2

Alle Behandlungen wurden wie im vorigen Beispiel, aber mit Einsatz einer 20 %igen Fluorkohlenstoffemulsion durchgeführt. Der Hämoglobingehalt wurde dreifach im Vergleich zu Anfangswerten abgesenkt, wobei durchschnittlich 65-70 Vol.-% des Blutes ersetzt wurden. Das Volumen des entnommenen Bluts und des injizierten Blutersatzmittels betrug jeweils 12,25 ml für das Gewicht von 250 g. In der Versuchsgruppe überlebten alle Tiere und in der Kontrollgruppe starben 5 Tiere.

### Versuch 3

Alle Behandlungen wurden wie im Beispiel 1 durchgeführt. 5 Tiere jeder Gruppe wurden aber nach 6 Stunden, einem Tag und 3 Tagen seit dem Blutersatz getötet. Die Lebermitochondrien wurden getrennt, und ein phosphorylierendes Atmen wurde registriert. In der Kontrollgruppe wurde eine rasche Unterdrückung der Atmungsgeschwindigkeit und ein Phosphorylieren bei einer Oxidation des NADabhängigen Substrats und Sukzinats von durchschnittlich über 50 % beobachtet, wodurch eine starke lschämieverletzung von Mitochondrien charakterisiert wird. In der Versuchsgruppe wurde eine 40 %ige Aktivierung des phosphorylirenden Atmens nach 6 Stunden seit dem Blutersatz beobachtet, die einen Tag lang erhalten blieb und höchstens 25 % nach 3 Tage seit dem Blutersatz betrug. Solche Änderungen charakterisieren erhalten gebliebene Lebermitochondrien, die an Hypoxie und nicht an Ischämie litten.

### Versuch 4

Dieser Versuch behandelte die Erhaltung von Nieren bei Hunden nach einem hämorrhagischen Schock. Die Nierenerhaltung wurde anhand einer Nierenwiederbelebung nach einer Nierentransplantation dem Tier mit beiden entfernten Nieren (die Untersuchung wurde nach Sondergenehmigung des Ethikausschusses des Gesundheitsministeriums durchgeführt) sowie anhand einer Bewertung der Adenylnukleotiden und des Laktat- und Pyruvatgehalts in den Nieren nach einer Stunde seit dem Blutersatz bestimmt. Untersucht wurden 10 Hunde mit je 5 Hunden in jeder Gruppe.

Untersuchungsvorgehensweise: dem Hund mit einem Gewicht von 20 kg wurden unter einer Intubationsnarkose mit gesteuerter Beatmung 400 ml Blut aus der Oberschenkelschlagader strahlweise entnommen, wodurch eine rasche Druckabnahme (bis 50-60 mm QS), eine verdoppelte Herzkontraktion und eine erhöhte Laktatkonzentration im Plasma bis 20mmol eintrat. Nach einer Stunde der Blutentnahme wurde dem Tier ein Blutersatzmittel, dessen Menge um 15 Vol.-% des Blutverlusts überschritt, zugeführt, nämlich eine 10 %ige Kohlenstoffemulsion nach Beispiel 1 mit einem Albuminzusatz bis 3,5 % (wie im Beispiel 14) in der Versuchsgruppe und der Plasmaexpander Polygluzin in der Kontrollgruppe. Nach einer weiteren Stunde wurden die Tiere getötet und die Nieren entnommen. Eine wurde zur Transplantation und die andere zur Untersuchung des Energieaustausches des Nierengewebes eingesetzt.

In der Kontrollgruppe nahm das Verhältnis von ATP zu ADP dreifach und die Energieladung ([ATP] + ½[ADP])/([ATP]+[ADP]+[AMP]) bis 0,45 ab. In der Versuchsgruppe (Blutersatz durch Fluorkohlenstoffemulsion) wurde das Verhältnis von ATP zu ADP höchstens zweifach und Energieladung bis auf 0,65-0,70 abgesenkt. Das Verhältnis von Laktat zu Pyruvat im Nierengewebe stieg in der Kontrollgruppe bis 25-30 und in der Versuchsgruppe höchstens bis 6.

In allen Fällen wurde ein Harnablassen bei den Empfängertieren mit Transplantatnieren derjenigen Hunde, die mit der Fluorkohlenstoffemulsion behandelt wurden, sofort nach dem Blutstromeinschluss des Transplantats beobachtet. In der Kontrollgruppe wurde die Entwicklung einer Reperfusionsverletzung mit raschem Gewebeödem und vollständigem Blutflussstillstand (Nierentod) in 2 Fällen aus 5 beobachtet. In drei Fällen der Kontrollgruppe wurde der Blutfluss in der Transplantatniere wiederhergestellt. Ein Harnablassen wurde erst nach einigen Stunden beobachtet.

Diese Daten weisen nach, dass die Behandlung eines hämorragischen Schocks bei Hunden mit einem Einsatz der Fluorkohlenstoffemulsion gemäß der Erfindung einen besseren Schutz der Organe vor einer Ischämie- und einer nachfolgenden Reperfusionsverletzung gewährleistet.

### Versuch 5

Dieser Versuch behandelte den Einsatz der nach Beispiel 2 hergestellten Fluorkohlenstoffemulsion zur Erhaltung perfundierter Kaninchenherzen. Vor dem Einsatz (1-2 Stunden) wurden 400 ml Fluorkohlenstoffemulsion mit 200 ml isotonische Lösung von Krebs-Henseleit im Verhältnis 2:1 gemischt. Zu 600 ml Gemisch wurden 80 ml einer 20 %igen Lösung von Serumalbumin zugegeben. Die Kontrollzusammensetzung für vergleichende Untersuchungen umfasste 600 ml Salzlösung mit einem Zusatz von 7,2 g Mannitol und 80 ml einer 20 %igen Albuminlösung. Diese Zusammensetzungen wurden als Perfusionsmilieu zur Erhaltung des Kaninchenherzens eingesetzt. Eine Langendorff-Perfusion wurde kreislaufweise bei 37° C durchgeführt. Registriert wurde die Zeit, in der die Häufigkeit und die Amplitude der Herzkontraktion erhalten blieben. Für die Kontrolle und Versuche wurden je 8 Herzen verwendet. Beim Einsatz der Perfusionsflüssigkeit auf Fluorkohlenstoffbasis blieb die Kontraktionsfähigkeit des isolierten Kaninchenherzens mindestens 6-8 Stunden erhalten. Bei der Perfusion mit der Kontrollzusammensetzung wurde jedoch eine starke Reduzierung der Häufigkeit und Amplitude der Kontraktion bis zum Herzstillstand beobachtet.

Zum Schluss soll erwähnt werden, dass die Vorteile der Emulsion gemäß der Erfindung gegenüber dem Prototyp und der der Erfindung am nächsten kommenden Emulsionen in Folgendem bestehen.

Die Rezeptur und das Herstellungsverfahren gemäß der Erfindung der Fluorkohlenstoffemulsionen gewährleisten eine fein verteilte, kalibrierte Emulsion mit vorgegebener Teilchengröße im Bereich zwischen 0,06 und 0,195 µm, die 2 bis 40 Vol.-% der Fluorkohlenstoffverbindungen enthält und mit einer Phospholipiddispersion in einer biologisch akzeptierbaren Wasser-Salz-Lösung stabilisiert wird. Es ist die Gewinnung einer hohen Feinheit und Mikrostruktur der Fluorkohlenstoffemulsionen bei 18monatiger Lagerung im nicht eingefrorenen Zustand gezeigt worden, wodurch ermöglicht wird, eine hohe Biokompatibilität, die sich in einer niedrigen Reaktogenität äußert, zu gewinnen. Die entwickelten Emulsionen sind zu biomedizinischen Zwecken anwendbar, nämlich zum Ersatz massiver Blutverluste, zur Behandlung hämorragischer Schocks, zur Vorbeuge einer ischämischen Reperfusionsverletzung, zur Vorbereitung der Organe für Transplantationen und zur Perfusionserhaltung isolierter Organe. Die entwickelten Emulsionen besitzen stark ausgeprägte Sauerstofftransport- und rheologische Eigenschaften, die eine Vorbeuge und Beseitigung einer Ischämieverletzung sauerstoffabhängiger Mitochondrienfunktionen sowie eine Unterstützung des aeroben Energieaustausches in Geweben bei einem Blutersatz und eine Behandlung hämorragischer Schocks gewährleisten.

### Literaturverzeichnis

1. Zeitschrift des russischen Mendelejew-Chemieverbandes, 1985, Band 30, Nr. 4, S. 387-394.
2. J. G. Rieses et al. Physiologische Aktivität fluorhaltender Verbindungen (Versuche und klinische Untersuchungen), Sammlung wissenschaftlicher Arbeiten, Puschtschino, 1995, S. 73-90.
3. M. B. Berkos, Kurzgefasste Dissertation... Doktor medizinischer Wissenschaften, Leningrad, 1991, 24 S.
4. J. G. Rieses, Chem. Rev., 2001, V. 101, Nr. 9, S. 2797-2914.
5. RU-Patent 2162692, Kl. 7 A61 K31/02, 9/10, 1999
6. RU-Patent 2199311, Kl. 7 A61 K9/107, 31 /02, 2001
7. US-Patent Nr. 3 778 381, 1973
8. US-Patent Nr. 6 113 919, 2000
9. US-Patent Nr. 4 866 096, A61K31/025, 1989
10. US-Patent Nr. 5 374 624, A61K31/025, 1994
11. RU-Patent Nr. 2088217, 6 A61K9/10,31/02,1997
12. Biophysik, 1998, Band 33, Nr.1, S. 126-129
13. I. N. Kusnezowa, Kurzgefasste Dissertation... Doktor biologischer Wissenschaften, St.-Petersburg, 1999, 38 S.
14. Chemisch-pharmazeutische Zeitschrift, 1987, Nr. 12, S. 1498-1503
15. Zeitschrift für physische Chemie, 1993, Band 67, Nr. 9, S. 1884-1888
16. E. I. Majewskij, Kurzgefasste Dissertation...Doktor med. Wissenschaften, Moskau, 1998, 36 S.

## Patentansprüche

1. Fluorkohlenstoffemulsion für medizinische Zwecke, die schnellausscheidende Fluorkohlenstoffverbindungen, wie Perfluordekalin oder Perfluoroctylbromid, einen Fluorkohlenstoffzusatz und ein Phospholipid umfasst,
**dadurch gekennzeichnet,**
**dass** als schnell ausscheidende Komponente eine Zusammensetzung aus Perfluordekalin und Perfluoroktylbromid eingesetzt wird, in der der Fluorkohlenstoffzusatz ein Gemisch aus perfluorierten, tertiären Aminen darstellt und das Phospholipid als Dispersion in einem Wasser-Salz-Milieu verwendet wird.

2. Emulsion nach Anspruch 1,
**dadurch gekennzeichnet,**
**dass** sie 2-40 Vol.-% an Fluorkohlenstoffverbindungen enthält.

3. Emulsion nach Anspruch 1,
**dadurch gekennzeichnet,**
**dass** die Zusammensetzung aus schnell ausscheidender Fluorkohlenstoffverbindungen Perfluordekalin und Perfluoroctylbromid im Verhältnis zwischen 10:1 und 1:10 enthält.

4. Emulsion nach Anspruch 1,
**dadurch gekennzeichnet,**
**dass** der Fluorkohlenstoffzusatz 1 bis 50 % des Summengehalts der Zusammensetzung aus den schnell ausscheidenden Fluorkohlenstoffverbindungen enthält.

5. Emulsion nach Anspruch 1,
**dadurch gekennzeichnet,**
**dass** das Gemisch aus perfluorierten, tertiären Aminen ein Gemisch aus Perfluortripropylamin und seinen Koprodukten, nämlich cis- und trans-Isomere von Perfluor-1-propyl-3,4-dimethylpirrolidon und Perfluor-1-propyl-4-methylpiperidin, enthält.

6. Emulsion nach Anspruch 1 und 5,
**dadurch gekennzeichnet,**
**dass** das Gemisch aus perfluorierten, tertiären Aminen zusätzlich Perfluor-N-methylzyklohexylpiperidin und seine Koprodukte enthält.

7. Emulsion nach Anspruch 1,
**dadurch gekennzeichnet,**
**dass** sie eine Dispersion aus Phospholipiden im Wasser-Salz-Milieu in einer Konzentration von 0,2 bis 5 Gew.-% enthält.

8. Emulsion nach Anspruch 1,
**dadurch gekennzeichnet,**
**dass** die Dispersion aus Phospholipiden im Wasser-Salz-Milieu Ei- oder Sojaphospholipide bzw. ein Gemisch dieser Lipide enthält.

9. Emulsion nach Anspruch 1,
**dadurch gekennzeichnet,**
**dass** die Dispersion aus Phospholipiden im Wasser-Salz-Milieu als Adjuvans pflanzliches Öl in einer Menge von 1-15 % des Summengehalts der Phospholipide enthält.

10. Emulsion nach Anspruch 9,
**dadurch gekennzeichnet,**
**dass** als Adjuvans Sojaöl dient.

11. Emulsion nach Anspruch 9,
**dadurch gekennzeichnet,**
**dass** als Adjuvans Sonnenblumenkernöl dient.

12. Emulsion nach Anspruch 9,
**dadurch gekennzeichnet,**
**dass** als Adjuvans Rizinusöl dient.

13. Emulsion nach Anspruch 9,
**dadurch gekennzeichnet,**
**dass** als Adjuvans ein Gemisch aus den genannten Ölen im wirksamen Verhältnis in Form eines zwei- oder dreifachen Gemisches dient.

14. Emulsion nach Anspruch 1,
**dadurch gekennzeichnet,**
**dass** die Zusammensetzung des Wasser-Salz-Milieus Natrium- und Kaliumsalze von Chloriden und Phosphaten sowie das Monosacharid Mannitol in Injektionswasser enthält.

15. Emulsion nach Anspruch 1,
**dadurch gekennzeichnet,**
**dass** die Konzentration der Komponenten im Wasser-Salz-Milieu einen osmotischen Druck im Bereich von 100-350 mosmol/1 aufweist.

16. Emulsion nach einem der Ansprüche 1 bis 15,
**dadurch gekennzeichnet,**
**dass** die mittlere Teilchengröße 0,2 µm nicht überschreitet und in einem Bereich von 0,06-0,2 µm liegt.

17. Verfahren zur Herstellung einer Fluorkohlenstoffemulsion, das ein Homogenisieren unter Hochdruck umfasst,
**dadurch gekennzeichnet,**
**dass** es in mehreren Stufen durchgeführt wird, nämlich ein erste Stufe zur Herstellung einer Dispersion aus Phospholipiden in einem Wasser-Salz-Milieu, ein zweite Stufe zum Homogenisieren von Fluorkohlenstoffverbindungen in der Dispersion aus Phospholipiden, einer dritten Stufe zur Hitzesterilisation der fertigen Emulsion und eine vierte Stufe zur nachfolgenden Lagerung von mindestens 6 Monaten im nicht eingefrorenen Zustand bei einer Temperatur von +4° C.

18. Verfahren nach Anspruch 17,
**dadurch gekennzeichnet,**
**dass** die Dispersion aus Phospholipiden im Wasser-Salz-Milieu durch Homogenisieren bei einem Hochdruck von mindestens 100 atm mit nachfolgender Hitzesterilisation hergestellt wird.

19. Verfahren nach Anspruch 17,
**dadurch gekennzeichnet,**
**dass** die Fluorkohlenstoffverbindungen in der Dispersion aus Phospholipiden bei einem Druck von 300 bis 650 atm homogenisiert werden.

20. Verfahren nach Anspruch 17,
**dadurch gekennzeichnet,**
**dass** die Dispersion aus Phospholipiden bei 100° C sterilisiert wird.

21. Verfahren nach Anspruch 17,
**dadurch gekennzeichnet,**
**dass** die Fluorkohlenstoffemulsion bei 100° C sterilisiert wird.
